(19) Europäisches Patentamt
European Patent Office
Office européen des brevets

(11) **EP 4 306 099 A1**

(12) **EUROPÄISCHE PATENTANMELDUNG**

(43) Veröffentlichungstag:
**17.01.2024 Patentblatt 2024/03**

(21) Anmeldenummer: 23180585.4

(22) Anmeldetag: **21.06.2023**

(51) Internationale Patentklassifikation (IPC):
*A61K 6/16* (2020.01)      *A61K 6/17* (2020.01)
*A61K 6/64* (2020.01)      *A61K 6/76* (2020.01)
*A61K 6/77* (2020.01)      *A61K 6/78* (2020.01)
*A61K 6/887* (2020.01)

(52) Gemeinsame Patentklassifikation (CPC):
(C-Sets verfügbar)
**A61K 6/887; A61K 6/16; A61K 6/17; A61K 6/64;
A61K 6/76; A61K 6/77; A61K 6/78**      (Forts.)

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(30) Priorität: **07.07.2022   DE 102022116932**

(71) Anmelder: **VOCO GmbH
27472 Cuxhaven (DE)**

(72) Erfinder:
• **Barg, Andree
21762 Otterndof (DE)**
• **Oldenburger, Daniel
27476 Cuxhaven (DE)**
• **Ebert, Tobias
27476 Cuxhaven (DE)**
• **Plaumann, Manfred Thomas
27472 Cuxhaven (DE)**

(54) **VERFAHREN ZUR HERSTELLUNG DENTALER FRÄSROHLINGE**

(57)    Die vorliegende Erfindung betrifft Verfahren zur Herstellung dentaler Fräsrohlinge, worin anorganische Füllstoffpulver in einem ersten Schritt mit einem Silan oberflächenbeschichtet werden und in zweiten Schritt eine polymerisierbare Zusammensetzung auf der silanisierten Oberfläche adsorbiert wird. Durch anschließendes Verpressen und Polymerisation werden homogene Fräsrohlinge mit sehr guten mechanischen Eigenschaften erhalten.

EP 4 306 099 A1

(52) Gemeinsame Patentklassifikation (CPC): (Forts.)

C-Sets
**A61K 6/887, C08L 33/08;**
**A61K 6/887, C08L 33/10;**
**A61K 6/887, C08L 33/26**

**Beschreibung**

Die vorliegende Erfindung betrifft ein Verfahren zur Herstellung kompositbasierter dentaler Fräsrohlinge sowie die nach diesen Verfahren hergestellten dentalen Fräsrohlinge.

Die Erfindung wird in den beigefügten Patentansprüchen definiert. Bevorzugte Aspekte der vorliegenden Erfindung ergeben sich überdies aus der nachfolgenden Beschreibung einschließlich der Beispiele.

Soweit für einen erfindungsgemäßen Aspekt (Verfahren, Zusammensetzung, Fräsrohling oder Kit) bestimmte Ausgestaltungen als bevorzugt bezeichnet werden, gelten die entsprechenden Ausführungen jeweils auch für die anderen Aspekte der vorliegenden Erfindung, *mutatis mutandis.* Bevorzugte individuelle Merkmale erfindungsgemäßer Aspekte (wie in den Ansprüchen definiert und/oder in der Beschreibung offenbart) sind miteinander kombinierbar und werden vorzugsweise miteinander kombiniert, sofern sich im Einzelfall für den Fachmann aus dem vorliegenden Text nichts anderes ergibt.

Der technische Fortschritt computergesteuerter Maschinen brachte es mit sich, dass Fräsmaschinen entwickelt wurden, die in der Lage sind, in kürzester Zeit und mit minimalem Aufwand prothetische Versorgungen in nicht gekannter Genauigkeit herzustellen. Vor diesem Hintergrund entwickelte sich die sogenannte "digitale Zahnmedizin". Sie ist heutzutage in der Zahnmedizintechnik von überragender Bedeutung.

Gefräst wurden anfangs ausschließlich keramische oder metallische Materialien, doch im Zuge der immer besser der natürlichen Zahnhartsubstanz angepassten dentalen Kompositmaterialien, wurde auch diese Substanzklasse für eine Verwendung als Fräsrohling interessant.

Dentale Fräsrohlinge auf Kompositbasis sind aus dem Stand der Technik bekannt.

Die DE 699 22 413 T2 beschreibt einen schneidfähigen Fräsrohling, der ein Polymerharz und ein feinverteiltes Füllstoffmaterial mit einem maximalen Partikeldurchmesser von weniger als 50 Mikrometer enthält. Die Harzphase der in diesem Dokument untersuchten dentalen Zusammensetzungen umfasst das System Bis-GMA (2,2-Bis[4-(2-hydroxy-3-methacryloyloxypropoxy)phenyl]propan) und TEGDMA (Triethylenglycoldimethacrylat), die Füllstoffphase enthält oberflächensilanisierte Kieselsäure und Glas.

Die EP 3 050 533 A1 offenbart dentale Harzblöcke, die als Harzphase UDMA/ TEGDMA (Urethandimethacrylat/ Triethylenglycoldimethacrylat) 80/20, UDMA/ Bis-MEPP/ HDP (Urethandimethacrylat/ 2,2-Bis(4-methacryloxypolyethoxy-phenyl)propan/ 2-Hydroxy-1,3-dimethacyloxypropan 60/20/20 und 50/30/20 sowie UDMA/ Bis-MEPP (Urethandimethacrylat/ 2,2-Bis(4-methacryloxypoly-ethoxyphenyl)propan 60/40 verwenden. Diese Harzzusammensetzungen werden mit anorganischen Füllstoffen zu radikalisch härtbaren Pasten in Gewichtsverhältnissen von Füllstoff zu Harz von 64:36 bis 70.8:29.2 vermischt und dann zu Harzblöcken mit Hilfe von BPO (Benzoylperoxid) thermisch polymerisiert.

Die DE 24 62 271 C2 beansprucht dentale Formkörper, enthaltend mindestens ein polymerisiertes Acrylat bzw. Methacrylat und einen silanisierten mikrofeinen anorganischen Füllstoff auf Siliciumdioxidbasis, die dadurch gekennzeichnet sind, dass sie als polymerisiertes Acrylat bzw. Methacrylat ein Polymerisat aus Bis-GMA oder einem anderen Derivat des Bisphenols A oder einem Reaktionsprodukt aus Hydroxyethylmethacrylaten und Diisocyanaten, gegebenenfalls zusammen mit Polymerisaten aus kurzkettigen Methacrylsäureestern und/oder aus difunktionellen Acryl- bez. Methacrylsäureestern und als anorganischen Füllstoff ausschließlich mikrofeines Siliciumdioxid mit einer Teilchengröße von 10 bis 400 nm und mit einer BET-Oberfläche von weniger als 200 $m^2$/g in einer Menge von 20 bis 80 Gewichtsprozent enthalten.

Die EP 2 623 065 B1 offenbart Rohlinge für eine Dentalfräse, die auf hohe mechanische Eigenschaften wie Biegefestigkeit und Glanzbeständigkeit abzielen. Erreicht werden diese Eigenschaften durch einen Rohling für eine Dentalfräse, der aus einem gehärteten Produkt einer aushärtbaren Zusammensetzung gebildet wird und umfasst: (a) ein polymerisierbares Monomer; (b) einen sphärischen anorganischen Füllstoff, der eine durchschnittliche Primärteilchengröße von nicht weniger als 0.1 $\mu$m und weniger als 1 $\mu$m aufweist und (c) einen anorganischen Ultrafeinteilchenaggregatfüllstoff, bestehend aus Aggregaten von anorganischen ultrafeinen Teilchen, die eine durchschnittliche Primärteilchengröße von 2 bis 50 nm aufweisen. Als Harzmatrix wird hier eine Monomermischung aus Bis-GMA/TEGDMA/Bis-MEPP (2,2-Bis[4-(2-hydroxy-3-methacryloyloxypropoxy)-phenyl]propan / Triethylenglycoldimethacrylat / 2,2-Bis(4-methacryloxypolyethoxy-phenyl)propan) im Gewichtsverhältnis 20/30/50 verwendet.

Die WO 2011/087832 A1 ist auf thermisch gehärtete Kompositrohlinge gerichtet, die signifikant verbesserte mechanische und ästhetische Eigenschaften aufweisen sollen. Die radikalisch härtbaren Zusammensetzungen enthalten Initiatoren, die thermisch zerfallen. Da der Zerfall bei höheren Temperaturen erfolgt, wird verhindert, dass Monomere unter normalen Verarbeitungsbedingungen vorzeitig gelieren und durch Abbauprozesse den Rohling verfärben.

Die WO 2016/140950 A1 beschreibt Kompositmaterialien, die im gehärteten Zustand auch als dentale Fräsrohlinge verwendet werden können. Die Zusammensetzungen enthalten eine härtbare Harzkomponente, Keramikfasern und Füllstoff in Form von Nanoclustern. Die Polymerisate sollen hochästhetische Restaurationen ergeben und ausgezeichnete Polierbarkeiten sowie Glanzretentionseigenschaften aufweisen. In einem Ausführungsbeispiel wird die Zusammensetzung einer Harzphase angegeben, deren Bestandteile Bis-GMA, TEGDMA, UDMA, BisEMA-6 (ethoxyliertes Bisphenol A dimethacrylat mit 6 Ethoxygruppen) und PEG600DM (Polyethylenglycoldimethacrylat mit einem Molekulargewicht der Polyethyleneinheiten von ca. 600 g/mol) in Gewichtsverhältnissen von 25/1.2/35/35/3.8 verwendet werden.

Die US 6,345,984 B2 betrifft Fräsrohlinge, wobei das Kompositmaterial einen partikulären Füllstoff in einem Größenbereich von ungefähr 0.1 bis 5.0 $\mu$m und kolloidales Silikat in einer Größe von 1 bis ungefähr 70 nm sowie eine Harzphase aus ethoxyliertem Bisphenol A-Dimethacrylat enthält und der Ethoxylierungsgrad von 1 bis 20, vorzugsweise von 2 bis 7 Mol Ethylenoxid/ mol BPA reicht. Der Fräsrohling besteht insgesamt aus ca. 20 bis ca. 30 Gew.-% aus einer organischen Matrix und aus ca. 65 bis ca. 85 Gew.-% partikulärem Füllstoff, wobei die organische Matrix aus ca. 65 bis ca. 90 Gew.-% ethoxyliertem BPA-Dimethacrylat und aus ca. 10 bis ca. 30 Gew.-% eines Methacrylatoligomers, beispielsweise eines Polycarbonatdimethacrylatkondensationsproduktes, besteht. Unter Berücksichtigung der in der US 6,345,984 B2 zitierten US 4,544,359 sieht die typische Zusammensetzung eines Fräsrohlings dann folgendermaßen aus: partikulärer Füllstoff (0.1 bis 5 $\mu$m) 65 bis 79 Gew.-%, kolloidales Silikat (1 bis 70 nm) 1 bis 5 Gew.-% und organische Matrix 20 bis 30 Gew.-%.

Die DE 198 23 530 B4 ist auf ein Dentalharzmaterial gerichtet, das zu einer Zahnprothese durch Fräsen geformt wird, das ein Acrylharzpolymer umfasst, das 20 bis 70 Gew.-% eines anorganischen Füllstoffs mit einer mittleren Teilchengröße von 10 bis 40 nm im Durchmesser enthält, 1 bis 40 Gew.-% eines Glaspulvers mit einer mittleren Teilchengröße von 0.1 - 5 $\mu$m im Durchmesser und 1 - 40 Gew.-% eines organisch-anorganischen Verbundfüllstoffes, der durch Mischen und Härten eines Gemisches aus einem ultrafeinen anorganischen Füllstoff mit einer mittleren Teilchengröße von 10 bis 40 nm im Durchmesser und einem Methacrylat- oder Acrylatmonomer mit mindestens einer ungesättigten Doppelbindung und Pulverisieren des gehärteten Gemisches derart, dass es eine mittlere Teilchengröße von 5 bis 50 $\mu$m im Durchmesser aufweist, hergestellt ist, wobei das Acrylharzpolymer eine Kombination aus einem Methacryl- oder Acrylatmonomer mit mindestens einer ungesättigten Doppelbindung und einem Wärmepolymerisationsinitiator umfasst. In Beispiel 5 wird die Kompositzusammensetzung eines Fräsrohlings beschrieben. Die Harzphase umfasst UDMA/Bis-MEPP in einem Gewichtsverhältnis von 5/20 und die Feststoffphase enthält 22 Gew.-% anorganischen Füllstoff (beispielsweise Aerosil mit einer mittleren Teilchengröße von 10 bis 40 nm), beispielsweise OX-50 (mit 40 nm Primärpartikelgröße), 23 Gew.-% Quarzglaspulver (mittlere Teilchengröße: 0.5 $\mu$m), 30 Gew.-% Bariumglaspulver (mittlere Teilchengröße: 0.5 $\mu$m).

Die EP 3 363 423 B1 betrifft Fräsrohlinge, die sich durch eine niedrige Wasseraufnahme, einen hohen E-Modul und durch eine hohe mechanische Festigkeit auszeichnen.

Die EP 3 685 798 B1 betrifft Fräsrohlinge, die sich durch eine niedrige Quellung auszeichnen und so ideal für die Herstellung von retentionssicheren Kronen und Brücken eignen.

Allen diesen Anmeldungen ist gemein, dass die Kompositblöcke aus Pasten hergestellt werden, die selbst auch als direkte Füllungsmaterialien geeignet wären und die auch die gleichen Vor- und Nachteile kompositbasierter Füllungsmaterialien mit sich bringen.

Die EP 3 287 118 A1 beschreibt ein Herstellungsverfahren für dentale Fräsrohlinge, bei dem anorganische Füllstoffe, Monomermatrix, Initiatoren und Farbmittel mittels Mahladditiv und Mahlgut intensiv miteinander vermahlen, anschließend getrocknet und dann verpresst werden. Nachteilig an diesem Verfahren ist der Einsatz von Mahlkugeln in Anwesenheit der Initiatoren in der Monomermatrix. Durch den lokal hohen Energieeintrag kann es lokal zu vorzeitigen kleinen Polymerisationszonen kommen, die dann Inhomogenitäten im fertigen Produkt darstellen.

Die EP 2 881 077 A1 zielt auf Fräsrohlinge ab, die ausgezeichnete mechanische Eigenschaften, eine ausgezeichnete Abriebfestigkeit und einen ausgezeichneten Oberflächenglanz aufweisen sollen. Diese Eigenschaften sollen hier verfahrenstechnisch erreicht werden. Im Gegensatz zu den herkömmlichen Herstellmethoden, bei denen das Kompositmaterial durch homogenes Mischen und Kneten einer härtbaren Monomermischung mit einem Füllstoff zu einer Paste verarbeitet wird, die eine bestimmte Fließfähigkeit aufweisen muss, um in einer Form zu einem Fräsrohling ausgehärtet zu werden, schlägt diese Anmeldung vor, den anorganischen Füllstoff heißzupressen, um ihn zu aggregieren und ihn schließlich mit dem härtbaren Harz zu infiltrieren. Das Harz soll so die Zwischenräume der Primärpartikel füllen und kann dann gehärtet werden. Dieses Verfahren soll die Herstellung von Rohlingen gestatten, bei denen die Füllstoffteilchen so dicht nebeneinander liegen, wie es beim konventionellen Herstellverfahren nicht möglich ist und somit erlauben, Fräsrohlinge mit einem besonders hohen Füllstoffgehalt zur Verfügung zu stellen.

Ein analoges Verfahren wird auch in den Anmeldungen JP 2017-109036 A, JP 2019-098073 A und JP 2019-098074 A der gleichen Anmelderin beschrieben.

Nachteilig an diesem Verfahren ist, dass der Füllstoff bereits vor der Harzinfiltration sehr stark kompaktiert ist und die Harzinfiltration nur von der Oberfläche erfolgt. Es besteht somit die Gefahr, dass das Harz nicht alle Zwischenräume insbesondere im Kern des Fräsrohlings vollständig erreicht und füllt.

Aufgabe der vorliegenden Erfindung war es somit ein Verfahren zur Verfügung zu stellen, mit dem sich dentale Fräsrohlinge herstellen lassen, die zum einen hochkompaktiert sind und zum anderen zuverlässig über die homogene Harz-Füllstoffverteilung konventioneller Fräsrohlinge verfügen, die aus Pasten hergestellt sind.

Gelöst wird diese Aufgabe durch ein Verfahren zur Herstellung eines dentalen Fräsrohlings umfassend die folgenden Schritte

i) Herstellen oder Bereitstellen eines anorganischen Füllstoffpulvers (F)

ii) zumindest teilweises Beschichten der Oberfläche des anorganischen Füllstoffpulvers (F) durch Reaktion mit einem Silan (S) der Formel

$PG\text{-}L\text{-}Si(R^1)_n(OR^2)_{(3\text{-}n)}$ worin

PG eine polymerisierbare Gruppe ist,

L eine Linkergruppe ist, die die polymerisierbare Gruppe PG an das Si-Atom bindet,

$R^1$ eine C1- bis C4-Alkylgruppe oder Phenylgruppe ist,

$R^2$ eine C1- bis C4-Alkylgruppe oder ein H-Atom ist und

n = 0, 1 oder 2 ist,

wobei ein oberflächenbeschichtetes Pulver ($F_{sil}$) erhalten wird,

iii) Adsorbieren einer flüssigen, polymerisierbaren Zusammensetzung (M), die in der Lage ist mit der polymerisierbaren Gruppe PG zu polymerisieren, auf der Oberfläche des in Schritt ii erhaltenen Füllstoffpulvers ($F_{sil}$), wobei ein oberflächenadsorbiertes Pulver ($F_{sil,ads}$) erhalten wird,

iv) Füllen des in Schritt iii erhaltenen Pulvers ($F_{sil,ads}$) in eine Form,

v) Verpressen des Pulvers ($F_{sil.ads}$) in der Form unter Druck und

vi) Polymerisation der polymerisierbaren Zusammensetzung M und der polymerisierbaren Gruppen PG.

Vorzugsweise erfolgt das Adsorbieren der flüssigen, polymerisierbaren Zusammensetzung (M) in Schritt iii durch energiearmes Mischen, bevorzugt in einem Taumel- oder Rotationsmischer.

Vorzugsweise erfolgt das Adsorbieren der flüssigen, polymerisierbaren Zusammensetzung (M) in Schritt iii ohne (energiereichen) Mahlprozess, bevorzugt ohne Zugabe von Mahladditiv und/oder Mahlmedium. Insbesondere ist es bevorzugt, keine flüchtigen organischen Lösungsmittel als Mahladditiv und/oder Achat- oder Zirkoniumdioxidkugeln als Mahlmedium zu zugeben.

In einer bevorzugten Ausführungsform handelt es sich dabei um ein entsprechendes Verfahren, wobei das Verfahren zusätzlich einen oder mehrere der folgenden Schritte umfasst

vii) Entformen des in Schritt vi erhaltenen Fräsrohlings und/oder

viii) Nachbearbeitung des in Schritt vi erhaltenen Fräsrohlings und/oder

ix) Befestigung eines Pins auf der Oberfläche des Fräsrohling zur Befestigung des Fräsrohlings in einer Fräsmaschine.

Überraschenderweise wurde dabei gefunden, dass durch das erfindungsgemäße Verfahren in den Schritten i bis iii ein Pulver erhalten wird, bei dem die polymerisierbare Zusammensetzung auf der Oberfläche adsorbiert ist, das sich aber wie ein Pulver handhaben und verarbeiten lässt. Insbesondere lässt es sich durch seine Rieselfähigkeit gut in Formen abfüllen (Schritt iv). So lassen sich zum Beispiel auch nacheinander unterschiedlich gefärbte Pulver in eine Form füllen. Außerdem lassen sich zwei (oder mehr) unterschiedlich gefärbte Pulver gleichzeitig unter mischen in eine Form füllen, wobei das Mischungsverhältnis während des Abfüllvorgangs, vorzugsweise kontinuierlich, variiert wird. Mit diesen beiden Verfahren lassen sich mehrfarbige dentale Fräsrohlinge erhalten, die entweder aus unterschiedlich gefärbten Schichten aufgebaut sind oder die einen kontinuierlichen Farbgradienten aufweisen. Diese Fräsrohlinge sind ideal in der Lage, den natürlichen Farbverlauf der Zähne nachzustellen.

Gleichzeitig lassen sich durch das Kompaktieren der oberflächenadsorbierten Pulver dentale Fräsrohlinge herstellen, die sehr gute mechanische Eigenschaften, insbesondere eine hohe Festigkeit, einen hohen Elastizitätsmodul und eine hohe Abrasionsstabilität aufweisen. Erreicht wird dies einerseits durch die homogene Harz-/Füllstoffverteilung die durch den Adsorptionsvorgang erzielt wird und andererseits durch die Minimierung von Fehlstellen, die durch das Kompaktieren erfolgt.

In einer vorteilhaften Ausgestaltung eines erfindungsgemäßen Verfahrens erfolgt das Verpressen in Schritt v, vorzugsweise uniaxial oder biaxial, bei einem Druck im Bereich von 400 bis 12000 bar, bevorzugt im Bereich von 2500 bis 10000 bar, besonders bevorzugt im Bereich von 4000 bis 10000 bar.

In einer vorteilhaften Ausgestaltung eines erfindungsgemäßen Verfahrens erfolgt die Polymerisation in Schritt vi, vorzugsweise isostatisch, bei einem Druck im Bereich von 50 bis 750 bar, bevorzugt im Bereich von 100 bis 600 bar, besonders bevorzugt im Bereich von 200 bis 500 bar, und/oder (vorzugsweise und) bei einer Temperatur im Bereich von 60 bis 160 °C, bevorzugt im Bereich von 80 bis 140 °C.

In einer weiteren vorteilhaften Ausgestaltung eines erfindungsgemäßen Verfahrens erfolgen die Schritte des Verpressens v und der Polymerisation vi gleichzeitig bei einer Temperatur im Bereich von 60 bis 160 °C, bevorzugt im Bereich von 80 bis 140 °C, durch

- uniaxialen oder biaxialen Druck im Bereich von 400 bis 12000 bar, bevorzugt im Bereich 2500 bis 10000 bar, besonders bevorzugt im Bereich von 4000 bis 10000 bar,

  oder

- isostatischen Druck im Bereich von 50 bis 750 bar, bevorzugt im Bereich von 100 bis 600 bar, besonders bevorzugt im Bereich von 200 bis 500 bar.

In einer vorteilhaften Ausgestaltung umfasst das in Schritt i hergestellte oder bereitgestellte anorganische Füllstoffpulver (F) einen oder mehrere anorganische Füllstoffe ausgewählt aus der Gruppe bestehend aus Bariumsilikatgläsern, Bariumaluminiumsilikatgläsern, Bariumborosilikatsilikatgläsern, Bariumboroaluminiumsilikatgläsern, Bariumborofluoroaluminiumsilikatgläsern, Strontiumsilikatgläsern, Strontiumaluminiumsilikatgläsern, Strontiumborosilikatgläsern, Strontiumboroaluminiumsilikatsilikatgläsern, Strontiumborofluoroaluminiumsilikatgläsern, Zirkonsilikatgläsern, Quarz, Cristobalit, pyrogener Kieselsäure und Ytterbiumfluorid, bevorzugt ausgewählt aus der Gruppe bestehend aus Bariumboroaluminiumsilikatsilikatgläsern, pyrogener Kieselsäure und Ytterbiumfluorid.

In einer vorteilhaften Ausgestaltung weist das anorganische Füllstoffpulver Feinen D50-Wert von 0.18 bis 10 $\mu$m, vorzugsweise von 0.4 bis 5 $\mu$m, auf.

In einer vorteilhaften Ausgestaltung umfasst das anorganische Füllstoffpulver Fein erstes anorganisches Füllstoffpulver F1 und ein zweites anorganisches Füllstoffpulver F2. Dabei können sich das erste anorganische Füllstoffpulver F1 und das zweite anorganische Füllstoffpulver F2 sowohl in ihrer chemischen Zusammensetzung als auch in ihrer Partikelgröße unterscheiden.

In einer weiteren vorteilhaften Ausgestaltung weist das erste anorganische Füllstoffpulver F1 einen D50-Wert von 0.4 bis 1.0 $\mu$m, vorzugsweise von 0.5 bis 0.9 $\mu$m, und das zweite anorganische Füllstoffpulver F2 einen D50-Wert von 1.2 bis 5.0 $\mu$m, vorzugsweise von 1.5 bis 4.0 $\mu$m, auf.

Unter "zumindest teilweises Beschichten" in Schritt ii wird verstanden, dass das anorganische Füllstoffpulver (F) Bestandteile enthält, die durch die Anwesenheit freier Si-OH-Gruppen in der Lage sind mit dem Silan PG-L-Si($R^1$)$_n$(O$R^2$)$_{(3-n)}$ zu reagieren. Darüber hinaus kann das anorganische Füllstoffpulver (F) aber auch weitere Bestandteile enthalten, die über keine freien Si-OH-Gruppen verfügen und somit nicht in der Lage sind dem Silan PG-L-Si($R^1$)$_n$(O$R^2$)$_{(3-n)}$ zu reagieren. Beispiele dafür sind Ytterbiumfluorid, das keine Si-OH-Gruppen aufweist oder hydrophobierte Kieselsäure, bei der die Si-OH-Gruppen bereits anderweitig umgesetzt sind. Vorzugsweise enthält das anorganische Füllstoffpulver (F) mindestens 50 Gew.-%, bevorzugt mindestens 70 Gew.-%, besonders bevorzugt mindestens 90 Gew.-% und ganz besonders bevorzugt 100 Gew.-% Bestandteile, die in der Lage sind mit dem Silan PG-L-Si($R^1$)$_n$(O$R^2$)$_{(3-n)}$ zu reagieren.

Das in Schritt ii eingesetzte Silan PG-L-Si($R^1$)$_n$(O$R^2$)$_{(3-n)}$ weist eine polymerisierbare Gruppe PG auf, die in der Lage ist mit der in Schritt iii adsorbierten polymerisierbaren Zusammensetzung (M) zu (co)polymerisieren. Vorzugsweise handelt es sich bei PG um eine (Meth)acryl- oder (Meth)acrylamidgruppe. Unter "(Meth)acryl" werden dabei sowohl "Methacryl" als auch "Acryl" verstanden.

In einer bevorzugten Ausführungsform ist die polymerisierbare Gruppe PG des Silans PG-L-Si($R^1$)$_n$(O$R^2$)$_{(3-n)}$ ausgewählt aus der Gruppe bestehend aus -OC(=O)CH=CH$_2$, -OC(=O)C(CH$_3$)=CH$_2$, -NHC(=O)CH=CH$_2$ und -NHC(=O)C(CH$_3$)=CH$_2$, bevorzugt -OC(=O)CH=CH$_2$ und -OC(=O)C(CH$_3$)=CH$_2$, besonders bevorzugt -OC(=O)C(CH$_3$)=CH$_2$.

In einer bevorzugten Ausführungsform ist die Linkergruppe L des Silans PG-L-Si($R^1$)$_n$(O$R^2$)$_{(3-n)}$ eine Alkylengruppe mit 1 bis 12 C-Atomen, die von einem Sauerstoffatom oder einer Urethangruppe unterbrochen sein kann.

Vorzugsweise ist L ausgewählt aus der Gruppe bestehend aus -(CH$_2$)$_p$-, -(CH$_2$)$_q$-OC(=O)NH-(CH$_2$)$_p$-und-(CH$_2$)$_q$-NHC(=O)O-(CH$_2$)$_p$-, mit p = 1 bis 8 und q = 2 bis 4.

In einer bevorzugten Ausführungsform handelt es sich bei dem Silan PG-L-Si($R^1$)$_n$(O$R^2$)$_{(3-n)}$ um ein Silan ausgewählt aus der Gruppe bestehend aus

H$_2$C=C(CH$_3$)C(=O)O-(CH$_2$)$_p$-Si(O$R^2$)$_3$,        H$_2$C=C(CH$_3$)C(=O)O-(CH$_2$)$_q$-NHC(=O)O-(CH$_2$)$_p$-Si(O$R^2$)$_3$      und

$H_2C=C(CH_3)C(=O)O-(CH_2)_q-OC(=O)NH-(CH_2)_p-Si(OR^2)_3$ worin $R^2$ eine C1- bis C4-Alkylgruppe ist, p = 1 bis 8 und q = 2 bis 4 ist.

In einer besonders bevorzugten Ausführungsform handelt es sich bei dem Silan $PG-L-Si(R^1)_n(OR^2)_{(3-n)}$ um ein Silan ausgewählt aus der Gruppe bestehend aus

$H_2C=C(CH_3)C(=O)O-(CH_2)-Si(OR^2)_3$, $\qquad$ $H_2C=C(CH_3)C(=O)O-(CH_2)_3-Si(OR^2)_3$,
$H_2C=C(CH_3)C(=O)O-(CH_2)_2-NHC(=O)O-(CH_2)_3-Si(OR^2)_3$ $\qquad$ und
$H_2C=C(CH_3)C(=O)O-(CH_2)_2-OC(=O)NH-(CH_2)_3-Si(OR^2)_3$ worin $R^2$ eine C1- bis C4-Alkylgruppe ist.

Ganz besonders bevorzugt handelt es sich bei dem Silan um 3-Methacryloxypropyltrimethoxysilan.

In einer bevorzugten Ausführungsform umfasst die flüssige, polymerisierbare Zusammensetzung (M)

A) ein, zwei oder mehrere polymerisierbare Monomere, vorzugsweise (Meth)acrylate und/oder (Meth)acrylamide,
B) einen Initiator oder ein Initiatorsystem,
C) optional anorganische, nicht-agglomerierte, nicht-aggregierte Partikel mit einer mittleren Partikelgröße von kleiner 80 nm und
D) optional Farbmittel.

Unter "(Meth)acryl" werden dabei sowohl "Methacryl" als auch "Acryl" verstanden.

Vorzugsweise bestehen die polymerisierbaren Monomere (A) aus (Meth)acrylaten.

Vorzugsweise umfassen die polymerisierbaren Monomere (A) ein oder mehrere Momomere ausgewählt aus der Gruppe bestehend aus Ethylenglycol-di(meth)acrylat, Diethylenglycoldi(meth)acrylat, Triethylenglycoldi(meth)acrylat, Tetraethylenglycoldi(meth)acrylat, Polyethylenglycoldi(meth)acrylat, Propylen-glycoldi(meth)acrylat, Dipropylenglycoldi(meth)acrylat, Tripropylenglycol-di(meth)acrylat, Tetrapropylenglycoldi(meth)acrylat, Polypropylenglycol-di(meth)acrylat, Neopentylglycoldi(meth)acrylat, 1,4-Butandioldi(meth)acrylat, 1,6-Hexandiol-di(meth)acrylat, 1,9-Nonandioldi(meth)acrylat, 1,10-Decandioldi-(meth)acrylat, 1,12-Dodecandioldi(meth)acrylat, Tricyclo[5.2.1.0$^{2,6}$]decan-3(4),8(9)-dimethanol-di(meth)acrylat, 2-Hydroxypropyl-1,3-di(meth)acrylat, 3-Hydroxypropyl-1,2-di(meth)acrylat, Urethandi(meth)acrylat, 7,7,9-Trimethyl-3,14-dioxa-4,13-dioxo-5,12-diazahexadecan-1,16-diyldi(meth)acrylat, 7,9,9-Trimethyl-3,14-dioxa-4,13-dioxo-5,12-diazahexadecan-1,16-diyldi(meth)acrylat, 1,5,5-Trimethyl-1-[(2-(meth)acryloyloxyethyl)carbamoylmethyl]-3-(2-(meth)acryloyl-oxyethyl)carbamoylcyclohexan, 1,3-Bis(3',8'-dioxo-2'-aza-4',7'-dioxa-decyl-9'-en)phenyl, 1,3-Bis(9'-methyl-3',8'-dioxo-2'-aza-4',7'-dioxa-decyl-9'-en)phenyl, 1,3-Bis(1',1'-dimethyl-3',8'-dioxo-2'-aza-4',7'-dioxa-decyl-9'-en)phenyl, 1,3-Bis(1',1',9'-trimethyl-3',8'-dioxo-2'-aza-4',7'-dioxa-decyl-9'-en)phenyl, 3(4),8(9)-Bis-(4',7'-dioxa-3',8'-dioxo-2'-aza-decyl-9'-en)tetrahydrodicyclopentadien, 3(4),8(9)-Bis-(4',7'-dioxa- 3',8'-dioxo-2'-aza-9'-methyl-decyl-9'-en)tetrahydrodicyclopentadien, Trimethylolpropandi(meth)acrylat, Trimethylolpropantri(meth)acrylat, ethoxyliertes Trimethylolpropantri(meth)acrylat, Ditrimethylolpropantetra(meth)acrylat, ethoxyliertes Ditrimethylolpropantetra(meth)acrylat, Pentaerythritoldi(meth)acrylat, Pentaerythritoltri(meth)acrylat, Pentaerythritoltetra(meth)acrylat, Dipenta-erythritoltetra(meth)acrylat, Dipentaerythritolpenta(meth)acrylat, Dipentaerythritol-hexa(meth)acrylat, 2,2-Bis[4-[3-(meth)acryloyloxy-2-hydroxypropoxy]phenyl]-propan, 2,2-Bis[4-[2-(meth)acryloyloxy-3-hydroxypropoxy]phenyl]propan, ethoxyliertes Bisphenol-A-di(meth)acrylat, propoxyliertes Bisphenol-A-di(meth)acrylat, 2,2-Bis[4-(meth)acryloyloxyethoxyphenyl]propan, 2,2-Bis[4-(meth)acryloyloxy-diethoxyphenyl]propan, 2,2-Bis[4-(meth)acryloyloxytriethoxyphenyl]propan, 2,2-Bis[4-(meth)acryloyloxytetraethoxyphenyl]propan, 2,2-Bis[4-(meth)acryloyloxy-pentaethoxyphenyl]-propan, 2,2-Bis[4-(meth)acryloyloxydipropoxyphenyl]propan, 2,2-Bis[4-(meth)acryloyloxyethoxyphenyl]-2-[4-(meth)acryloyloxydiethoxyphenyl]-propan, 2-[4-(Meth)acryloyloxydiethoxy-phenyl]-2-[4-(meth)acryloyloxytriethoxy-phenyl]propan, 2-[4-(Meth)acryloyloxdipropoxyphenyl]-2-[4-(meth)acryloyloxy-triethoxyphenyl]propan, 2-Hydroxyethyl(meth)acrylat, 2-Hydroxypropyl(meth)-acrylat, 3-Hydroxypropyl(meth)acrylat, 1,2-Dihydroxypropyl(meth)acrylat, 1,3-Dihydroxypropyl(meth)acrylat, Methyl(meth)acrylat, Ethyl(meth)acrylat, Propyl-(meth)acrylat, Butyl(meth)acrylat, Hexyl(meth)acrylat, 2-Ethylhexyl(meth)acrylat, Tetrahydrofurfuryl(meth)acrylat, Isobornyl(meth)acrylat, Lauryl(meth)acrylat, Cyclohexyl(meth)acrylat, (Octahydro-4,7-methano-1H-indenyl)methyl(meth)-acrylat, Benzyl(meth)acrylat und Phenoxyethyl(meth)acrylat, bevorzugt ausgewählt aus der Gruppe bestehend aus Triethylenglycoldi(meth)acrylat, Tetraethylenglycoldi(meth)acrylat, 1,6-Hexandiol¬di(meth)acrylat, 1,10-Decan-dioldi(meth)acrylat, 1,12-Dodecandioldi(meth)acrylat, Tricyclo[5.2.1.0$^{2,6}$]decan-3(4),8(9)-dimethanol-di(meth)acrylat, 2-Hydroxypropyl-1,3-di(meth)acrylat, 3-Hydroxypropyl-1,2-di(meth)acrylat, Urethandi(meth)acrylat, 7,7,9-Trimethyl-3,14-dioxa-4,13-dioxo-5,12-diazahexadecan-1,16-diyldi(meth)acrylat, 7,9,9-Trimethyl-3,14-dioxa-4,13-dioxo-5,12-diazahexadecan-1,16-diyldi(meth)acrylat, 1,5,5-Trimethyl-1-[(2-(meth)acryloyloxyethyl)carbamoylmethyl]-3-(2-(meth)acryloyloxy-ethyl)carbamoylcyclohexan, 1,3-Bis(3',8'-dioxo-2'-aza-4',7'-dioxa-decyl-9'-en)-phenyl, 1,3-Bis(9'-methyl-3',8'-dioxo-2'-aza-4',7'-dioxa-decyl-9'-en)phenyl, 1,3-Bis(1',1'-dimethyl-3',8'-dioxo-2'-aza-4',7'-dioxa-decyl-9'-en)phenyl, 1,3-Bis(1',1',9'-trimethyl-3',8'-dioxo-2'-aza-4',7'-dioxa-decyl-9'-en)phenyl, 3(4),8(9)-Bis-(4',7'-dioxa-3',8'-dioxo-2'-aza-decyl-9'-en)tetrahydrodicyclopentadien, 3(4),8(9)-Bis-(4',7'-dioxa- 3',8'-dioxo-2'-aza-9'-methyl-decyl-9'-en)tetrahydrodicyclopentadien, 2,2-Bis[4-[3-(meth)acryloyloxy-2-hydroxypropoxy]phenyl]propan, 2,2-Bis[4-[2-(meth)-acryloyloxy-3-hydroxypropoxy]phenyl]propan, ethoxyliertes Bisphenol-A-di(meth)acrylat, propoxyliertes Bisphenol-A-di(meth)acrylat, 2,2-Bis[4-(meth)-acryloyloxyethoxyphenyl]propan, 2,2-Bis[4-(meth)acryloyloxydiethoxy-phenyl]-propan, 2,2-Bis[4-(meth)acryloyloxytriethoxyphenyl]propan, 2,2-Bis[4-(meth)acryloyloxytetraethoxyphenyl]propan, 2,2-Bis[4-(meth)acryloyloxypenta-ethoxyphenyl]-propan, 2,2-Bis[4-(meth)acryloyloxydipropoxyphenyl]propan, 2,2-Bis[4-(meth)acryloyloxyethoxyphenyl]-2-[4-(meth)acryloyloxydiethoxyphenyl]-propan, 2-[4-(Meth)acryloyloxydiethoxy-phenyl]-2-[4-(meth)acryloyloxytriethoxy-phenyl]propan und 2-[4-(Meth)acryloyloxdipropoxyphenyl]-2-[4-(meth)acryloyloxy-triethoxyphenyl]propan.

Der Anteil an polymerisierbaren Monomeren (A) beträgt 39 bis 99 Gew.-%, bevorzugt 39 bis 79 Gew.-%, besonders bevorzugt 39 bis 59 Gew.-%, bezogen auf die Gesamtmasse der flüssigen, polymerisierbaren Zusammensetzung (M).

In einer bevorzugten Ausführungsform handelt es sich bei dem Initiator oder Initiatorsystem (B) um einen thermischen Initiator. Geeignete thermische Initiatoren sind Ketonperoxide, Peroxyketale, Hydroperoxide, Dialkylperoxide, Diacylperoxide, Peroxyester, Peroxydicarbonate und Azoverbindungen. Vorzugsweise ist (B) ausgewählt aus der Gruppe bestehend aus Dibenzoylperoxid, Bis(2,4-dichlorbenzoyl)peroxid, Dilauroylperoxid, Dicumyl-peroxid, tert.-Butylperbenzoat, Cumolhydroperoxid, 3,5,5-Trimethylhexansäure-tert.-butylperoxyester 2,2'-Azobisisobutyronitril, 2,2'-Azobis(2-methylbutyronitril), 2,2'-Azobis-2,4-dimethylvaleronitril, 2,2'-Azobis-1-cyclohexancabonitril und Dimethyl-2-2'-azobisisobutyrat. Bevorzugt ist (B) ausgewählt aus der Gruppe bestehend aus Dibenzoylperoxid, Bis(2,4-dichlorbenzoyl)peroxid, Dilauroylperoxid und 2,2'-Azobisisobutyronitril. Besonders bevorzugt ist (B) Dibenzoylperoxid.

Der Anteil an Initiatoren (B) beträgt 0.5 bis 5 Gew.-%, bevorzugt 0.5 bis 3.0 Gew.-%, besonders bevorzugt 1.0 bis 2.5 Gew.-%, bezogen auf die Gesamtmasse der flüssigen, polymerisierbaren Zusammensetzung (M).

Vorzugsweise ist die flüssige, polymerisierbare Zusammensetzung (M) frei von nichtpolymerisierbaren Lösungsmitteln, bevorzugt frei von Lösungsmitteln.

In einer bevorzugten Ausführungsform enthält die flüssige, polymerisierbare Zusammensetzung (M) anorganische, nicht-agglomerierte, nicht-aggregierte Partikel (C). Diese Partikel haben eine mittlere Partikelgröße von kleiner 80 nm. Unter mittlerer Partikelgröße wird hier der volumengewichtete D50-Wert verstanden, der sich durch DLS-Messung bestimmen lässt. Vorzugsweise weisen die anorganischen, nicht-agglomerierten, nicht-aggregierten Partikel (C) einen D50-Wert von 8 bis 80 nm, bevorzugt von 10 bis 60 nm auf.

Vorteilhafter Weise liegen die Nanopartikel monodispers und vollständig dispergiert vor.

Der Anteil an anorganischen, nicht-agglomerierten, nicht-aggregierten Partikeln (C) beträgt 0 bis 60 Gew.-%, bevorzugt 20 bis 60 Gew.-%, besonders bevorzugt 40 bis 60 Gew.-%, bezogen auf die Gesamtmasse der flüssigen, polymerisierbaren Zusammensetzung (M).

In einer bevorzugten Ausführungsform handelt es sich bei den anorganischen, nicht-agglomerierten, nicht-aggregierten Partikeln (C) um nanoskalige Kieselsäure. Solche Kieselsäuren sind zum Beispiel durch Sol-Gel-Verfahren herstellbar und sind kommerziell beispielsweise unter der Bezeichnung "NALCO COLLOIDAL SILICAS" (Nalco Chemical Co.), "Ludox colloidal silica" (Grace) oder "Highlink OG (Clariant) erhältlich.

Um eine gute Einbindung der Nanopartikel in die Polymermatrix zu gewährleisten sind die nanoskaligen Kieselsäure-Partikel vorzugsweise mit einem Silan oberflächenbeschichtet. Vorzugsweise wird dazu ein Silan der Formel $PG-L-Si(R^1)_n(OR^2)_{(3-n)}$ verwendet, wobei die Definitionen für PG, L, $R^1$, $R^2$ und n die gleichen sind, die bereits im Zusammenhang mit dem anorganischen Füllstoffpulvers (F) und dem Silan (S) weiter oben getroffen wurden. Für das Silan zur Oberflächenbeschichtung der Kieselsäure-Nanopartikel gelten die gleichen bevorzugten Ausgestaltungen die bereits oben für das Silan (S) beschrieben sind. Für die Oberflächenbeschichtung des anorganischen Füllstoffpulvers (F) und der anorganischen, nicht-agglomerierten, nicht-aggregierten Partikeln (C) auf Silica-Basis kann sowohl das gleiche Silan als auch zwei unterschiedliche Silane, die jeweils der Formel $PG-L-Si(R^1)_n(OR^2)_{(3-n)}$ entsprechen verwendet werden. Besonders bevorzugt ist aber auch hier die Verwendung von 3-Methacryloxypropyltrimethoxysilan.

In einer weiteren bevorzugten Ausführungsform handelt es sich bei den anorganischen, nicht-agglomerierten, nicht-aggregierten Partikeln (C) um nanoskaliges Ytterbiumfluorid ($YbF_3$).

Um eine gute Einbindung der Nanopartikel in die Polymermatrix zu gewährleisten sind die nanoskaligen Ytterbiumfluorid-Partikel vorzugsweise mit 10-[(2-Methylprop-2-enoyl)oxy]decyldihydrogenphosphat (MDP) oberflächenbeschichtet.

In einer bevorzugten Ausführungsform umfasst die flüssige, polymerisierbare Zusammensetzung (M) 40 bis 60 Gew.-% nicht-agglomerierte, nicht-aggregierte $SiO_2$- und/oder $YbF_3$-Partikel mit einem D50-Wert von 10 bis 60 nm.

Um eine möglichst naturgetreue, zahnähnliche Restauration zu ermöglichen enthält ein erfindungsgemäß hergestellter Fräsrohling Farbmittel.

Bei den Farbmittel handelt es sich um anorganische Farbpigmente, organische Farbpigmente und Farbstoffe. Für einen zahnähnlichen Eindruck werden weiße, gelbe, orange, rote, braune und schwarze Farbmittel eingesetzt.

Bevorzugt umfassen die Farbmittel anorganische Farbpigmente ausgewählt aus der Gruppe bestehend aus Eisenoxid, Titandioxid, Bariumsulfat und Aluminiumoxid.

Je nach chemischer Art der Farbmittel sowie bei Farbpigmenten nach deren Partikelgröße, können die Farbmittel in unterschiedlichen Schritten des erfindungsgemäßen Verfahrens zugegeben werden.

Lösliche organische Farbstoffe werden vorteilhaft in der flüssigen, polymerisierbaren Zusammensetzung (M) gelöst und mit dieser in Schritt iii zugegeben.

Auch anorganische oder organische Farbpigmente mit einer kleinen Partikelgröße, insbesondere mit einer Partikelgröße kleiner 80 nm, können ebenfalls gut in der flüssigen, polymerisierbaren Zusammensetzung (M) dispergiert und mit dieser in Schritt iii zugegeben werden.

Anorganische oder organische Farbpigmente können aber auch bereits in Schritt i dem anorganischen Füllstoffpulver (F) zugegeben und mit diesem gemischt werden.

Weiterhin ist es auch möglich anorganische oder organische Farbpigmente entweder beim Silanisierungsschritt ii oder beim Adsorptionsschritt iii zuzugeben.

Erfindungsgemäß ist demnach ein Verfahren zur Herstellung eines dentalen Fräsrohlings, wobei in einem der Schritte i, ii oder iii ein oder mehrere Farbmittel zugegeben werden oder wobei die flüssige, polymerisierbare Zusammensetzung (M) Farbmittel (D) umfasst.

Erfindungsgemäß ist auch ein Verfahren zur Herstellung eines dentalen Fräsrohlings, bei dem die Schritte i bis iv schichtweise wiederholt werden und die Pulverschichten in Schritt v gemeinsam verpresst werden oder bei dem die Schritte i bis v schichtweise wiederholt werden, wobei jeweils die einzelnen Schichten durch unterschiedliche Farbmittel und/oder durch eine unterschiedliche Menge an Farbmitteln unterschiedlich eingefärbt sind.

Erfindungsgemäß ist auch ein Verfahren zur Herstellung eines dentalen Fräsrohlings, bei dem parallel jeweils in den Schritten i bis iii zwei (oder mehr) unterschiedlich gefärbte oberflächenadsorbierte Pulver $F_{sil,ads,1}$ und $F_{sil,ads,2}$ (und ggf. weitere $F_{sil,ads,x}$) hergestellt werden und in Schritt iv unter Mischen in eine Form abgefüllt werden, wobei das Mischungsverhältnis von $F_{sil,ads,1}$ zu $F_{sil,ads,2}$ (und ggf. zu weiteren $F_{sil,ads,x}$) während des Abfüllen geändert, bevorzugt kontinuierlich geändert, wird.

Erfindungsgemäß ist auch ein Verfahren zur Herstellung eines dentalen Fräsrohlings, das zwischen den Schritten v und vi einen Schritt v-a umfasst, bei dem der verpresste Formkörper in eine Farbmittellösung getaucht wird. Vorzugsweise umfasst die Farbmittellösung Farbmittel und polymerisierbare Monomere sowie optional Initiatoren. Die Farbmittel sind vorzugsweise die bereits als Farbmittel (D) beschrieben Farbmittel. Die polymerisierbaren Monomere sind vorzugsweise die bereits als Monomere (A) beschrieben Monomere. Die Initiatoren sind vorzugsweise die bereits als Initiatoren (B) beschriebenen Initiatoren.

In einer bevorzugten Ausführungsform umfasst die flüssige, polymerisierbare Zusammensetzung (M)

A) in einer Menge von 39 bis 99 Gew.-%, bevorzugt 39 bis 79 Gew.-%, besonders bevorzugt 39 bis 59 Gew.-%,
B) in einer Menge von 0.5 bis 5 Gew.-%, bevorzugt 0.5 bis 3.0 Gew.-%, besonders bevorzugt 1.0 bis 2.5 Gew.-%,
C) in einer Menge von 0 bis 60 Gew.-%, bevorzugt 20 bis 60 Gew.-%, besonders bevorzugt 40 bis 60 Gew.-% und
D) in einer Menge von 0 bis 1 Gew.-%, bevorzugt 0 bis 0.5 Gew.-%, besonders bevorzugt 0.0001 bis 0.1 Gew.-%,

jeweils bezogen auf die Gesamtmasse der flüssigen, polymerisierbaren Zusammensetzung (M).

Durch das erfindungsgemäße Verfahren lassen sich dentale Fräsrohlinge mit einem besonders hohen Füllstoffgehalt herstellen.

In einer bevorzugten Ausführungsform liegt das Massenverhältnis von oberflächenbeschichtetem Pulver ($F_{sil}$) zur flüssigen, polymerisierbaren Zusammensetzung (M) im Bereich von 3:1 bis 10:1, bevorzugt im Bereich von 4:1 bis 9:1 und besonders bevorzugt im Bereich von 5:1 bis 8:1.

In einer bevorzugten Ausführungsform liegt der Gesamtfüllstoffgehalt, die Summe der Massenanteile des oberflächenbeschichteten Pulvers ($F_{sil}$) und ggf. der anorganischen, nicht-agglomerierten, nicht-aggregierten Partikel (C), im Bereich von 80 bis 95 Gew.-%, bevorzugt im Bereich von 83 bis 94 Gew.-% und besonders bevorzugt im Bereich von 85 bis 93 Gew.-%, jeweils bezogen auf die Gesamtmasse des Fräsrohlings.

Erfindungsgemäß ist auch ein dentaler Fräsrohling, der nach einem erfindungsgemäßen Verfahren hergestellt wurde, insbesondere ein dentaler Fräsrohling hergestellt durch Verpressen und Polymerisation eines anorganischen Füllstoffpulvers ($F_{sil,ads}$), auf dessen Oberfläche sich

eine Beschichtung mit einem Silan (S) $PG\text{-}L\text{-}Si(R^1)_n(OR^2)_{(3-n)}$ worin PG eine polymerisierbare Gruppe ist, L eine Linkergruppe ist, die die polymerisierbare Gruppe PG an das Si-Atom bindet, $R^1$ eine C1- bis C4-Alkylgruppe oder Phenylgruppe ist, $R^2$ eine C1- bis C4-Alkylgruppe oder ein H-Atom ist und n = 0, 1 oder 2 ist,
und
eine darauf adsorbierte flüssige, polymerisierbare Zusammensetzung (M) umfassend

(A) ein, zwei oder mehrere polymerisierbare Monomere, vorzugsweise (Meth)acrylate und/oder (Meth)acrylamide,
(B) einen Initiator oder ein Initiatorsystem, bevorzugt ein thermischer Initiator,
(C) optional anorganische, nicht-agglomerierte, nicht-aggregierte Partikel mit einer mittleren Partikelgröße von kleiner 80 nm und

(D) optional Farbmittel

befindet.

Die weiter oben für das Herstellungsverfahren beschriebenen vorteilhaften Ausgestaltungen in Bezug auf die einzelnen Bestandteile (anorganisches Pulver F, Silan S, polymerisierbare Gruppe PG, Linkergruppe L, $R^1$, $R^2$, n, p, q, polymerisierbare Zusammensetzung M, polymerisierbare Monomere A, Initiatoren B, Nanofüllstoffe C, Farbmittel D) gelten auch für den Fräsrohling als bevorzugt. Vorzugsweise werden dabei bevorzugte Merkmale mit einander kombiniert.

Vorteilhafter Weise werden erfindungsgemäße Fräsrohlinge oder die nach den erfindungsgemäßen Verfahren hergestellten Fräsrohlinge in Form von Kits zusammengestellt.

Erfindungsgemäß ist auch ein Kit umfassend mehrere Fräsrohlinge, hergestellt nach dem erfindungsgemäßen Verfahren, wobei die Fräsrohlinge

- die gleiche monochrome Farbe aufweisen oder
- unterschiedliche monochrome Farben aufweisen oder
- den gleichen schichtweisen Farbverlauf aufweisen oder
- den gleichen kontinuierlichen Farbverlauf aufweisen.

Erfindungsgemäß ist auch ein Kit umfassend

- einen oder mehrere Fräsrohlinge, hergestellt nach dem erfindungsgemäßen Verfahren,
- ein Konditionierungsmittel für aus den Fräsrohlingen gefertigte indirekte Restaurationen und
- ein Befestigungsmaterial zur Befestigung von aus den Fräsrohlingen gefertigte indirekte Restaurationen.

Die Erfindung wird nachfolgend anhand von Beispielen näher erläutert:

Abkürzungen (eingesetzte Substanzen):

| | |
|---|---|
| F1: | Barium-Aluminium-Borosilikat-Glas (D50 0.8 $\mu$m / D25 0.5 $\mu$m / D75 1.0 $\mu$m) |
| F2: | Barium-Aluminium-Borosilikat-Glas (D50 2.7 $\mu$m / D25 1.4 $\mu$m / D75 6.1 $\mu$m) |
| F3: | Barium-Aluminium-Borosilikat-Glas (D50 0.18 $\mu$m; GM27884 NF180, Schott) |
| Bis-GMA: | 2,2-Bis[4-methacryloyloxypolyethoxyphenyl]propan (CAS 1565-94-2) |
| Bis-EMA2,6: | Ethoxyliertes Bisphenol A-Dimethacrylat mit durchschnittlich 2,6 Ethylenoxideinheiten (CAS 41637-38-1) |
| UDMA: | 7,7,9(7,9,9)-Trimethyl-4,13-dioxo-3,14-dioxa-5,12-diazahexadecan-1,16-diyldimethacrylat (CAS 72869-86-4) |
| TCDDMA: | Bis(methacryloyloxymethyl)tricyclo[5.2.1.0$^{2,6}$]decan (CAS 43048-08-4) |
| TEGDMA: | Triethylengylcoldimethacrylat (CAS 109-16-0) |
| HDDMA: | 1,6-Hexanedioldimethacrylat (CAS 6606-59-3) |
| MPS: | 3-Methacryloxypropyltrimethoxysilan (CAS 2530-85-0) |
| MOS: | 8-Methacryloxyoctyltrimethoxysilan (CAS 122749-49-9) |
| BPO: | Dibenzoylperoxid (CAS 94-36-0) |
| TPO: | 2,4,6-Trimethylbenzoyldiphenylphosphinoxid (CAS 75980-60-8) |
| BHT: | 2,6-Di-*tert.*-butyl-4-methylphenol (CAS 128-37-0 |
| DEPT: | N,N-Dihydroxyethyl-4-methylanilin (CAS 3077-12-1) |

3-Punkt-Biegefestigkeit (BF): Die Biegefestigkeit wurde analog DIN EN ISO 6872:2009 (7.3.2) bei einer Stützweite von 12 mm und einem Auflagerollendurchmesser von 2 mm bestimmt. Dazu wurden aus den Komposit-Blöcken Probekörper mit einer Breite von 4 mm, einer Dicke von 1,2 mm und einer Länge von 15 mm mit einer Hochgeschwindigkeitssäge (IsoMet 4000, Buehler) hergestellt, entgratet, geschliffen und poliert. Die Proben wurden mit einer Traversengeschwindigkeit von 1 mm/min bis zum Bruch belastet und die 3-Punkt-Biegefestigkeit entsprechend der unter 7.3.2.4.1 angegebenen Formel berechnet.

Elastizitätsmodul (E): Der Elastizitätsmodul wurde analog der Berechnung in ADA Spec. No. 27:1993 (7.8.4.2) als Steigung der Spannung-Dehnungs-Kurve der 3-Punkt-Biegefestigkeitsmessung im linear-elastischen Bereich ermittelt.

$$E = \frac{3\,L}{4\,b\,h^3}\frac{\Delta F}{\Delta d}$$

(L: Stützweite; b: Probenbreite; h: Probendicke; $\Delta d$: Deformation im linear-elastischen Bereich; $\Delta F$: Kraftänderung bei einer Deformation $\Delta d$)

Glührückstand (G): Zur Bestimmung des Glührückstands wurde jeweils ein Kompositblock gemahlen und der Mahlrückstand anschließend homogenisiert. Etwas 0.5 g des Mahlrückstands wurden in einem Porzellantiegel für 2 Stunden auf 575 °C erhitzt. Der Glührückstand ergibt sich

$$G = \frac{m_{Auswaage}}{m_{Einwaage}} \times 100\%$$

($m_{Einwaage}$: Masse des eingewogenen, gemahlenen Blocks vor Erhitzen; $m_{Auswaage}$: Masse des gemahlenen Blocks nach Erhitzen)

Beispiel 1a (Füllstoff F1$_{sil}$-1a)

100.0 g Füllstoff F1 werden in 250 ml Ethanol dispergiert. Es werden 4.0 g MPS und 2.5 g Wasser zugegeben und zwei Stunden bei Raumtemperatur gerührt. Anschließend wird das Lösungsmittel im Vakuum entfernt und der Rückstand für drei Stunden bei 90 °C getrocknet.

Beispiel 1b (Füllstoff F2$_{sil}$-1b)

100.0 g Füllstoff F2 werden in 250 ml Ethanol dispergiert. Es werden 4.0 g MPS und 2.5 g Wasser zugegeben und zwei Stunden bei Raumtemperatur gerührt. Anschließend wird das Lösungsmittel im Vakuum entfernt und der Rückstand für drei Stunden bei 90 °C getrocknet.

Beispiel 1c (Füllstoff F3$_{sil}$-1c)

100.0 g Füllstoff F3 (GM27884, NF180) werden in 250 ml Ethanol dispergiert. Es werden 4.0 g MPS und 2.5 g Wasser zugegeben und zwei Stunden bei Raumtemperatur gerührt. Anschließend wird das Lösungsmittel im Vakuum entfernt und der Rückstand für drei Stunden bei 90 °C getrocknet.

Beispiel 1d (Füllstoff F1$_{sil}$-1d)

100.0 g Füllstoff F1 werden in 250 ml Ethanol dispergiert. Es werden 4.0 g MOS und 2.5 g Wasser zugegeben und zwei Stunden bei Raumtemperatur gerührt. Anschließend wird das Lösungsmittel im Vakuum entfernt und der Rückstand für drei Stunden bei 90 °C getrocknet.

Beispiel 1e (Füllstoff F2$_{sil}$-1e)

100.0 g Füllstoff F2 werden in 250 ml Ethanol dispergiert. Es werden 4.0 g MOS und 2.5 g Wasser zugegeben und zwei Stunden bei Raumtemperatur gerührt. Anschließend wird das Lösungsmittel im Vakuum entfernt und der Rückstand für drei Stunden bei 90 °C getrocknet.

Beispiel 2a (Harz-2a)

500.0 g Organosilicasol MIBK-SD-L (30%ig, 40 nm), 60.0 g Bis-EMA2,6, 37.5 g UDMA und 37.5 g TCDDMA werden für eine Stunde bei Raumtemperatur gerührt, bis sich eine homogene Lösung gebildet hat. Anschließend wird das Lösungsmittel im Vakuum entfernt. Dann werden 3.0 g BPO zugegeben und es wird für weitere zwei Stunden bei Raumtemperatur gerührt, bis sich das BPO vollständig gelöst hat.

Beispiel 2b (Harz-2b)

100.0 g UDMA, 25.0 g TEGDMA, 0.25 g BPO, 0.30 g BHT und 0.325 g DEPT werden für zwei Stunden bei Raumtemperatur gerührt, bis sich die Feststoffe vollständig gelöst haben.

Beispiel 2c (Harz-2c)

50.0 g Bis-GMA, 50.0 g HDDMA, 0.5 g TPO und 1.0 g BPO werden für zwei Stunden bei Raumtemperatur gerührt, bis sich die Feststoffe vollständig gelöst haben.

Beispiel 3a (Füllstoff $F_{sil,ads}$-3a)

Zu 70.5 g Füllstoff $F2_{sil}$-1b werden 29.5 g Harz-2a in vier Portionen zugegeben und für 16 Stunden bei Raumtemperatur an einem Taumelmischer TURBULA T2F (Willy A. Bachofen GmbH, Deutschland) mit 72 U/min gemischt.

Beispiel 3b (Füllstoff $F_{sil,ads}$-3b)

Zu 76.0 g Füllstoff $F2_{sil}$-1b werden 24.0 g Harz-2a in vier Portionen zugegeben und für 16 Stunden bei Raumtemperatur an einem Taumelmischer TURBULA T2F mit 72 U/min gemischt.

Beispiel 3c (Füllstoff $F_{sil,ads}$-3c)

Zu 13.0 g Füllstoff $F1_{sil}$-1a und 57.5 g Füllstoff $F2_{sil}$-1b werden 29.5 g Harz-2a in vier Portionen zugegeben und für 16 Stunden bei Raumtemperatur an einem Taumelmischer TURBULA T2F mit 72 U/min gemischt.

Beispiel 3d (Füllstoff $F_{sil,ads}$-3d)

Zu 14.0 g Füllstoff $F1_{sil}$-1a und 62.0 g Füllstoff $F2_{sil}$-1b werden 24.0 g Harz-2a in vier Portionen zugegeben und für 16 Stunden bei Raumtemperatur an einem Taumelmischer TURBULA T2F mit 72 U/min gemischt.

Beispiel 3e (Füllstoff $F_{sil,ads}$-3e)

Zu 13.0 g Füllstoff $F1_{sil}$-1d und 57.5 g Füllstoff $F2_{sil}$-1e werden 29.5 g Harz-2a in vier Portionen zugegeben und für 16 Stunden bei Raumtemperatur an einem Taumelmischer TURBULA T2F mit 72 U/min gemischt.

Beispiel 3f (Füllstoff $F_{sil,ads}$-3f)

Zu 14.0 g Füllstoff $F1_{sil}$-1d und 62.0 g Füllstoff $F2_{sil}$-1e werden 24.0 g Harz-2a in vier Portionen zugegeben und für 16 Stunden bei Raumtemperatur an einem Taumelmischer TURBULA T2F mit 72 U/min gemischt.

Beispiel 3g (Füllstoff $F_{sil,ads}$-3g)

Zu 75.0 g Füllstoff $F2_{sil}$-1b werden 25.0 g Harz-2b in vier Portionen zugegeben und für 16 Stunden bei Raumtemperatur an einem Taumelmischer TURBULA T2F mit 72 U/min gemischt.

Beispiel 4a

11.0 g Füllstoff $F_{sil,ads}$-3a werden in eine Stahlform (D 16 mm) gefüllt. Anschließend wird an einer Laborpresse (MP250, Maassen GmbH, Deutschland) für 5 Minuten bei 9750 bar uniaxial gepresst. Der Rohling wird vorsichtig aus der Form entnommen und isostatisch bei 250 bar und folgendem Temperaturprogramm (20 °C - 2 °C/min - 120 °C (30 min) - 5 °C/min - 20 °C) polymerisiert.

Beispiele 4b bis 4g

Analog zu Beispiel 4a werden aus den Pulvern $F_{sil,ads}$-3b bis $F_{sil,ads}$-3g Kompositblöcke hergestellt.

Beispiel 4h

11.0 g Füllstoff $F_{sil,ads}$-3c werden in eine Stahlform (D 16 mm) gefüllt. Anschließend wird an einer Laborpresse (MP250, Maassen GmbH, Deutschland) für 5 Minuten bei 4875 bar uniaxial gepresst. Der Rohling wird vorsichtig aus der Form entnommen und isostatisch bei 250 bar und folgendem Temperaturprogramm (20 °C - 2 °C/min - 120 °C (30 min) - 5 °C/min - 20 °C) polymerisiert.

Tabelle 1 (Beispiel 4)

| Beispiel | 4a | 4b | 4c | 4d |
|---|---|---|---|---|
| $F1_{sil}$-1a | - | - | 13.0 | 14.0 |
| $F2_{sil}$-1b | 70.5 | 76.0 | 57.5 | 62.0 |
| Harz-2a | 29.5 | 24.0 | 29.6 | 24.0 |
| Biegefestigkeit | 269 | 281 | 291 | 295 |
| E-Modul | 13.8 | 14.2 | 18.5 | 19.1 |
| Aussehen | homogen | homogen | homogen | homogen |

Tabelle 1 (Beispiel 4; Fortsetzung)

| Beispiel | 4e | 4f | 4g | 4h |
|---|---|---|---|---|
| $F1_{sil}$-1a | 13.0 | 14.0 | - | 13.0 |
| $F2_{sil}$-1b | 57.5 | 62.0 | 75.0 | 57.5 |
| Harz-2a | 29.6 | 24.0 | 25.0 | 29.6 |
| Biegefestigkeit | 283 | 289 | 258 | 290 |
| E-Modul | 18.5 | 19.1 | 14.3 | 18.4 |
| Aussehen | homogen | homogen | homogen | homogen |

Vergleichsbeispiel 5a

70.5 g Füllstoff $F2_{sil}$-1b und 29.5 g Harz-2a werden für 30 Minuten bei 50 U/min an einem Laborkneter (PC Laborsystem, Magden CH) geknetet, wobei nach ca. 10 Minuten ein Pastenumschlag von pulverförmig zu pastös erfolgt. Abschließend wird am Laborkneter für weitere 15 Minuten bei 50 U/min und -0,85 bar entlüftet. Zur Herstellung der Kompositblöcke wird die Paste in Formen (D 16 mm; h 20 mm) gefüllt. Anschließend wird isostatisch bei 250 bar und folgendem Temperaturprogramm (20 °C - 2 °C/min - 120 °C (30 min) - 5 °C/min - 20 °C) polymerisiert.

Vergleichsbeispiel 5b

76.0 g Füllstoff F2$_{sil}$-1b und 24.0 g Harz-2a werden für 30 Minuten bei 50 U/min an einem Laborkneter (PC Laborsystem, Magden CH) geknetet. Bei diesem Pulver/Flüssigkeits-Verhältnis erfolgt kein Pastenumschlag von pulverförmig zu pastös mehr. Eine Weiterverarbeitung der Kompositpaste zu einem Kompositblock ist daher nicht möglich.

Vergleichsbeispiel 5c

13.0 g Füllstoff F1$_{sil}$-1a, 57.5 g Füllstoff F2$_{sil}$-1b und 29.5 g Harz-2a werden für 30 Minuten bei 50 U/min an einem Laborkneter (PC Laborsystem, Magden CH) geknetet, wobei nach ca. 10 Minuten ein Pastenumschlag von pulverförmig zu pastös erfolgt. Abschließend wird am Laborkneter für weitere 15 Minuten bei 50 U/min und -0,85 bar entlüftet. Zur Herstellung der Kompositblöcke wird die Paste in Formen (D 16 mm; h 20 mm) gefüllt. Anschließend wird isostatisch bei 250 bar und folgendem Temperaturprogramm (20 °C - 2 °C/min - 120 °C (30 min) - 5 °C/min - 20 °C) polymerisiert.

Vergleichsbeispiel 5d

14.0 g Füllstoff F1$_{sil}$-1a, 62.0 g Füllstoff F2$_{sil}$-1b und 24.0 g Harz-2a werden für 30 Minuten bei 50 U/min an einem Laborkneter (PC Laborsystem, Magden CH) geknetet. Bei diesem Pulver/Flüssigkeits-Verhältnis erfolgt kein Pastenumschlag von pulverförmig zu pastös mehr. Eine Weiterverarbeitung der Kompositpaste zu einem Kompositblock ist daher nicht möglich.

Tabelle 2 (Vergleichsbeispiel 5)

| Vergleichsbeispiel | 5a | 5b | 5c | 5d |
|---|---|---|---|---|
| F1$_{sil}$-1a | - | - | 13.0 | 14.0 |
| F2$_{sil}$-1b | 70.5 | 76.0 | 57.5 | 62.0 |
| Harz-2a | 29.5 | 24.0 | 29.6 | 24.0 |
| Biegefestigkeit | 221 | n.b.* | 274 | n.b.* |
| E-Modul | 13.4 | n.b.* | 18.3 | n.b.* |
| Aussehen | homogen | n.b.* | homogen | n.b.* |
| * nicht bestimmt, da sich kein Block herstellen ließ | | | | |

Vergleichsbeispiel 6a (Füllstoff V6a)

70.5 g Füllstoff F2, 29.5 g Harz-2a, 200.0 g Ethanol und 50.0 g Zirconia-Mahlkugeln (LUVOBEAD YZB, 1.0-1.2 mm) werden für 60 Minuten in einer Rührwerksmühle gemahlen. Anschließend erfolgt eine Vortrocknung für 3 Stunden bei 50 °C und eine Haupttrocknung für weitere 3 Stunden bei 50 °C.

Vergleichsbeispiel 6b (Füllstoff V6b)

76.0 g Füllstoff F2, 24.0 g Harz-2a, 200.0 g Ethanol und 50.0 g Zirconia-Mahlkugeln (LUVOBEAD YZB, 1.0-1.2 mm) werden für 60 Minuten in einer Rührwerksmühle gemahlen. Anschließend erfolgt eine Vortrocknung für 3 Stunden bei 50 °C und eine Haupttrocknung für weitere 3 Stunden bei 50 °C.

Vergleichsbeispiel 6c (Füllstoff V6c)

70.5 g Füllstoff F2$_{sil}$-1b, 29.5 g Harz-2a, 200.0 g Ethanol und 50.0 g Zirconia-Mahlkugeln (LUVOBEAD YZB, 1.0-1.2 mm) werden für 60 Minuten in einer Rührwerksmühle gemahlen. Anschließend erfolgt eine Vortrocknung für 3 Stunden bei 50 °C und eine Haupttrocknung für weitere 3 Stunden bei 50 °C.

Vergleichsbeispiel 6d (Füllstoff V6d)

76.0 g Füllstoff F2$_{sil}$-1b, 24.0 g Harz-2a, 200.0 g Ethanol und 50.0 g Zirconia-Mahlkugeln (LUVOBEAD YZB, 1.0-1.2 mm) werden für 60 Minuten in einer Rührwerksmühle gemahlen. Anschließend erfolgt eine Vortrocknung für 3 Stunden bei 50 °C und eine Haupttrocknung für weitere 3 Stunden bei 50 °C.

Vergleichsbeispiel 6e (Füllstoff V6e)

75.0 g Füllstoff F2, 25.0 g Harz-2b, 100.0 g Ethanol und 100.0 g Zirconia-Mahlkugeln (LUVOBEAD YZB, 1.0-1.2 mm) werden für 90 Minuten in einer Rührwerksmühle gemahlen. Anschließend erfolgt eine Vortrocknung für 3 Stunden bei 40 °C und eine Haupttrocknung für weitere 3.5 Stunden bei 40 °C.

Vergleichsbeispiel 7a

11.0 g Füllstoff V6a werden in eine Stahlform (D 16 mm) gefüllt. Anschließend wird an einer Laborpresse (MP250, Maassen GmbH, Deutschland) für 5 Minuten bei 10 MPa uniaxial gepresst. Der Rohling wird vorsichtig aus der Form entnommen und isostatisch bei 130 MPa und 150 °C für 90 Minuten polymerisiert.

Vergleichsbeispiel 7b bis 7e

Analog zu Vergleichsbeispiel 7a werden aus den Pulvern V6b bis V6e Kompositblöcke hergestellt.

Tabelle 3 (Vergleichsbeispiel 7)

| Vergleichsbeispiel | 7a | 7b | 7c | 7d | 7e |
|---|---|---|---|---|---|
| F2 | 70.5 | 76.0 | - | - | 75.0 |
| F2$_{sil}$-1b | - | - | 70.5 | 76.0 | - |
| Harz-2a | 29.5 | 24.0 | 29.5 | 24.0 | - |
| Harz-2b | - | - | - | - | 25.0 |
| Biegefestigkeit | 203 | 211 | 212 | 216 | 202 |
| E-Modul | 10.7 | 11.5 | 10.8 | 11.9 | 10.6 |
| Aussehen | inhomogen | inhomogen | inhomogen | inhomogen | inhomogen |

Die Prüfkörper der Biegeprüfung wurden an den Bruchstellen mikroskopisch untersucht. Die Prüfkörper wiesen eine Vielzahl von Inhomogenitäten auf, die darauf hindeuten, dass zum Teil eine vorzeitige Polymerisation während des Mahlvorgangs oder während des Trocknungsvorgangs stattgefunden hat.

Vergleichsbeispiel 8a

11.0 g Füllstoff V6a werden in eine Stahlform (D 16 mm) gefüllt. Anschließend wird an einer Laborpresse (MP250, Maassen GmbH, Deutschland) für 5 Minuten bei 9750 bar uniaxial gepresst. Der Rohling wird vorsichtig aus der Form entnommen und isostatisch bei 250 bar und folgendem Temperaturprogramm (20 °C - 2 °C/min - 120 °C (30 min) - 5 °C/min - 20 °C) polymerisiert.

Vergleichsbeispiele 8b bis 8e

Analog zu Vergleichsbeispiel 8a werden aus den Pulvern V6b bis V6e Kompositblöcke hergestellt.

Tabelle 4 (Vergleichsbeispiel 8)

| Vergleichsbeispiel | 8a | 8b | 8c | 8d | 8e |
|---|---|---|---|---|---|
| F2 | 70.5 | 76.0 | - | - | 75.0 |
| F2$_{sil}$-1b | - | - | 70.5 | 76.0 | - |
| Harz-2a | 29.5 | 24.0 | 29.5 | 24.0 | - |
| Harz-2b | - | - | - | - | 25.0 |
| Biegefestigkeit | 205 | 218 | 215 | 229 | 205 |

(fortgesetzt)

| Vergleichsbeispiel | 8a | 8b | 8c | 8d | 8e |
|---|---|---|---|---|---|
| E-Modul | 10.6 | 11.8 | 10.9 | 12.1 | 10.8 |
| Aussehen | inhomogen | inhomogen | inhomogen | inhomogen | inhomogen |

Auch hier wiesen die Prüfkörper eine Vielzahl von Inhomogenitäten auf, die darauf hindeuten, dass zum Teil eine vorzeitige Polymerisation während des Mahlvorgangs oder während des Trocknungsvorgangs stattgefunden hat.

Vergleichsbeispiel 9a

5.5 g Füllstoff $F2_{sil}$-1b in eine Stahlform (D 16 mm) gefüllt. Anschließend wird an einer Laborpresse (MP250, Maassen GmbH, Deutschland) für 3 Minuten bei 68.6 MPa uniaxial gepresst. Der Rohling wird vorsichtig aus der Form entnommen, in 10 ml Harz-2a eingetaucht und für 12 Stunden unter Lichtausschluss bei Raumtemperatur stehen gelassen. Anschließend wird unter Beibehaltung des Tauchzustands der Druck reduziert und die Entgasung durchgeführt (10 hPa, 10 Minuten). Der Rohling wird aus dem Harz entnommen, auf eine Glasplatte gelegt und für 5 Minuten in einer Lichtbox (Individo Light Box, VOCO GmbH, Deutschland) photopolymerisiert. Anschließend erfolgt eine thermische Polymerisation bei 130 °C für 20 Minuten.

Vergleichsbeispiel 9b

11.0 g Füllstoff $F2_{sil}$-1b in eine Stahlform (D 16 mm) gefüllt. Anschließend wird an einer Laborpresse (MP250, Maassen GmbH, Deutschland) für 3 Minuten bei 68.6 MPa uniaxial gepresst. Der Rohling wird vorsichtig aus der Form entnommen, in 20 ml Harz-2a eingetaucht und für 12 Stunden unter Lichtausschluss bei Raumtemperatur stehen gelassen. Anschließend wird unter Beibehaltung des Tauchzustands der Druck reduziert und die Entgasung durchgeführt (10 hPa, 10 Minuten). Der Rohling wird aus dem Harz entnommen, auf eine Glasplatte gelegt und für 5 Minuten in einer Lichtbox (Individo Light Box, VOCO GmbH, Deutschland) photopolymerisiert. Anschließend erfolgt eine thermische Polymerisation bei 130 °C für 20 Minuten.

Vergleichsbeispiel 9c

11.0 g Füllstoff $F2_{sil}$-1b in eine Stahlform (D 16 mm) gefüllt. Anschließend wird an einer Laborpresse (MP250, Maassen GmbH, Deutschland) für 3 Minuten bei 68.6 MPa uniaxial gepresst. Der Rohling wird vorsichtig aus der Form entnommen, in 20 ml Harz-2a eingetaucht und für 4 Tage unter Lichtausschluss bei Raumtemperatur stehen gelassen. Anschließend wird unter Beibehaltung des Tauchzustands der Druck reduziert und die Entgasung durchgeführt (10 hPa, 10 Minuten). Der Rohling wird aus dem Harz entnommen, auf eine Glasplatte gelegt und für 5 Minuten in einer Lichtbox (Individo Light Box, VOCO GmbH, Deutschland) photopolymerisiert. Anschließend erfolgt eine thermische Polymerisation bei 130 °C für 20 Minuten.

Vergleichsbeispiel 9d

5.5 g Füllstoff $F3_{sil}$-1c in eine Stahlform (D 16 mm) gefüllt. Anschließend wird an einer Laborpresse (MP250, Maassen GmbH, Deutschland) für 3 Minuten bei 68.6 MPa uniaxial gepresst. Der Rohling wird vorsichtig aus der Form entnommen, in 10 ml Harz-2c eingetaucht und für 12 Stunden unter Lichtausschluss bei Raumtemperatur stehen gelassen. Anschließend wird unter Beibehaltung des Tauchzustands der Druck reduziert und die Entgasung durchgeführt (10 hPa, 10 Minuten). Der Rohling wird aus dem Harz entnommen, auf eine Glasplatte gelegt und für 5 Minuten in einer Lichtbox (Individo Light Box, VOCO GmbH, Deutschland) photopolymerisiert. Anschließend erfolgt eine thermische Polymerisation bei 130 °C für 20 Minuten.

Vergleichsbeispiel 9e

11.0 g Füllstoff $F3_{sil}$-1c in eine Stahlform (D 16 mm) gefüllt. Anschließend wird an einer Laborpresse (MP250, Maassen GmbH, Deutschland) für 3 Minuten bei 68.6 MPa uniaxial gepresst. Der Rohling wird vorsichtig aus der Form entnommen, in 20 ml Harz-2c eingetaucht und für 12 Stunden unter Lichtausschluss bei Raumtemperatur stehen gelassen. Anschließend wird unter Beibehaltung des Tauchzustands der Druck reduziert und die Entgasung durchgeführt (10 hPa, 10 Minuten). Der Rohling wird aus dem Harz entnommen, auf eine Glasplatte gelegt und für 5 Minuten in einer Lichtbox (Individo Light Box, VOCO GmbH, Deutschland) photopolymerisiert. Anschließend erfolgt eine thermische Polymerisation bei 130 °C für 20 Minuten.

Vergleichsbeispiel 9f

11.0 g Füllstoff $F3_{sil}$-1c in eine Stahlform (D 16 mm) gefüllt. Anschließend wird an einer Laborpresse (MP250, Maassen GmbH, Deutschland) für 3 Minuten bei 68.6 MPa uniaxial gepresst. Der Rohling wird vorsichtig aus der Form entnommen, in 20 ml Harz-2c eingetaucht und für 4 Tage unter Lichtausschluss bei Raumtemperatur stehen gelassen. Anschließend wird unter Beibehaltung des Tauchzustands der Druck reduziert und die Entgasung durchgeführt (10 hPa, 10 Minuten). Der Rohling wird aus dem Harz entnommen, auf eine Glasplatte gelegt und für 5 Minuten in einer Lichtbox (Individo Light Box, VOCO GmbH, Deutschland) photopolymerisiert. Anschließend erfolgt eine thermische Polymerisation bei 130 °C für 20 Minuten.

Tabelle 5 (Vergleichsbeispiel 9)

| Vergleichsbeispiel | 9a | 9b | 9c | 9d | 9e | 9f |
|---|---|---|---|---|---|---|
| Füllstoff | $F2_{sil}$-1b | | | $F3_{sil}$-1c | | |
| Harz | Harz-2a | | | Harz-2c | | |
| Glührückstand | 81.5% | 70.3% | 81.3% | 81.8% | 70.7% | 81.5% |
| Biegefestigkeit | 170 | 150 | 168 | 173 | 151 | 169 |
| E-Modul | 12.4 | 9.9 | 12.3 | 12.8 | 10.1 | 12.7 |

Es zeigt sich, dass insbesondere für Blöcke in klinisch relevanten Dimensionen eine ausreichend lange Infiltrationszeit notwendig ist, um eine möglichst vollständige Sättigung mit dem Harz zu erzielen. Gleichwohl ist es von außen nur schwer möglich, den Infiltrationsgrad während der Eintauchzeit zu bestimmen, um eine vorzeitige Entnahme zu vermeiden.

Die vorliegende Erfindung lässt sich anhand der folgenden Aspekte zusammenfassen.

1. Verfahren zur Herstellung eines dentalen Fräsrohlings umfassend die folgenden Schritte

i) Herstellen oder Bereitstellen eines anorganischen Füllstoffpulvers (F),

ii) zumindest teilweises Beschichten der Oberfläche des anorganischen Füllstoffpulvers (F) durch Reaktion mit einem Silan (S) der Formel

$PG\text{-}L\text{-}Si(R^1)_n(OR^2)_{(3-n)}$ worin
PG eine polymerisierbare Gruppe ist,
L eine Linkergruppe ist, die die polymerisierbare Gruppe PG an das Si-Atom bindet,
$R^1$ eine C1- bis C4-Alkylgruppe oder Phenylgruppe ist,
$R^2$ eine C1- bis C4-Alkylgruppe oder ein H-Atom ist und
n = 0, 1 oder 2 ist,
wobei ein oberflächenbeschichtetes Pulver ($F_{sil}$) erhalten wird,

iii) Adsorbieren einer flüssigen, polymerisierbaren Zusammensetzung (M), die in der Lage ist mit der polymerisierbaren Gruppe PG zu polymerisieren, auf der Oberfläche des in Schritt ii erhaltenen Füllstoffpulvers ($F_{sil}$), wobei ein oberflächenadsorbiertes Pulver ($F_{sil,ads}$) erhalten wird,
iv) Füllen des in Schritt iii erhaltenen Pulvers ($F_{sil,ads}$) in eine Form,
v) Verpressen des Pulvers ($F_{sil,ads}$) in der Form unter Druck und
vi) Polymerisation der polymerisierbaren Zusammensetzung M und der polymerisierbaren Gruppen PG.

2. Verfahren zur Herstellung eines dentalen Fräsrohlings nach Aspekt 1, wobei das Verfahren zusätzlich einen oder mehrere der folgenden Schritte umfasst

vii) Entformen des in Schritt vi erhaltenen Fräsrohlings und/oder
viii) Nachbearbeitung des in Schritt vi erhaltenen Fräsrohlings und/oder
ix) Befestigung eines Pins auf der Oberfläche des Fräsrohlings zur Befestigung des Fräsrohlings in einer Fräsmaschine.

3. Verfahren zur Herstellung eines dentalen Fräsrohlings nach einem der vorangehenden Aspekte, wobei Schritt iii durch energiearmes Mischen erfolgt.

4. Verfahren zur Herstellung eines dentalen Fräsrohlings nach einem der vorangehenden Aspekte, wobei Schritt iii durch Mischen in einem Taumel- oder Rotationsmischer erfolgt.

5. Verfahren zur Herstellung eines dentalen Fräsrohlings nach einem der vorangehenden Aspekte, wobei Schritt iii ohne Mahlprozess durchgeführt wird und kein Mahladditiv zugegeben wird.

6. Verfahren zur Herstellung eines dentalen Fräsrohlings nach einem der vorangehenden Aspekte, wobei Schritt iii ohne Mahlprozess durchgeführt wird und kein Lösungsmittel als Mahladditiv zugegeben wird.

7. Verfahren zur Herstellung eines dentalen Fräsrohlings nach einem der vorangehenden Aspekte, wobei Schritt iii ohne Mahlprozess durchgeführt wird und kein Mahlmedium zugegeben wird.

8. Verfahren zur Herstellung eines dentalen Fräsrohlings nach einem der vorangehenden Aspekte, wobei Schritt iii ohne Mahlprozess durchgeführt wird und keine Achat- oder Zirkoniumkugeln als Mahlmedium zugegeben wird.

9. Verfahren zur Herstellung eines dentalen Fräsrohlings nach einem der vorangehenden Aspekte, wobei Schritt iii ohne Mahlprozess durchgeführt wird und kein Mahladditiv und kein Mahlmedium zugegeben wird.

10. Verfahren zur Herstellung eines dentalen Fräsrohlings nach einem der vorangehenden Aspekte, wobei in einem der Schritte i, ii oder iii ein oder mehrere Farbmittel zugegeben werden oder wobei die flüssige, polymerisierbare Zusammensetzung (M) Farbmittel (D) umfasst.

11. Verfahren zur Herstellung eines dentalen Fräsrohlings nach einem der vorangehenden Aspekte, das zwischen den Schritten v und vi einen Schritt v-a umfasst, bei dem der verpresste Formkörper in eine Farbmittellösung getaucht wird.

12. Verfahren zur Herstellung eines dentalen Fräsrohlings nach einem der vorangehenden Aspekte, wobei die Farbmittellösung Farbmittel (D), polymerisierbare Monomere (A), vorzugsweise (Meth)acrylate und/oder (Meth)acrylamide, und optional Initiatoren umfasst.

13. Verfahren zur Herstellung eines dentalen Fräsrohlings nach einem der vorangehenden Aspekte, bei dem die Schritte i bis iv schichtweise wiederholt werden und die Pulverschichten in Schritt v gemeinsam verpresst werden.

14. Verfahren zur Herstellung eines dentalen Fräsrohlings nach einem der vorangehenden Aspekte, bei dem die Schritte i bis v schichtweise wiederholt werden, wobei jeweils die einzelnen Schichten durch unterschiedliche Farbmittel und/oder durch eine unterschiedliche Menge an Farbmitteln unterschiedlich eingefärbt sind.

15. Verfahren zur Herstellung eines dentalen Fräsrohlings nach einem der vorangehenden Aspekte, bei dem parallel jeweils in den Schritten i bis iii zwei oder mehr unterschiedlich gefärbte oberflächenadsorbierte Pulver $F_{sil,ads,1}$ bis $F_{sil,ads,x}$ hergestellt werden und in Schritt iv unter Mischen in eine Form abgefüllt werden, wobei das Mischungsverhältnis von $F_{sil,ads,1}$ zu $F_{sil,ads,2}$ und gegebenenfalls zu weiteren $F_{sil,ads,x}$ während des Abfüllen geändert, bevorzugt kontinuierlich geändert, wird.

16. Verfahren zur Herstellung eines dentalen Fräsrohlings nach einem der vorangehenden Aspekte, wobei das Verpressen in Schritt v, vorzugsweise uniaxial oder biaxial, bei einem Druck im Bereich von 400 bis 12000 bar, bevorzugt im Bereich von 2500 bis 10000 bar, besonders bevorzugt im Bereich von 4000 bis 10000 bar, erfolgt.

17. Verfahren zur Herstellung eines dentalen Fräsrohlings nach einem der vorangehenden Aspekte, wobei die Polymerisation in Schritt vi, vorzugsweise isostatisch, bei einem Druck im Bereich von 50 bis 750 bar, bevorzugt im Bereich von 100 bis 600 bar, besonders bevorzugt im Bereich von 200 bis 500 bar, und/oder bei einer Temperatur im Bereich von 60 bis 160 °C, bevorzugt im Bereich von 80 bis 140 °C, erfolgt.

18. Verfahren zur Herstellung eines dentalen Fräsrohlings nach einem der vorangehenden Aspekte, wobei die Schritte des Verpressens v und der Polymerisation vi gleichzeitig bei einer Temperatur im Bereich von 60 bis 160 °C, bevorzugt im Bereich von 80 bis 140 °C, erfolgen durch

- uniaxialen oder biaxialen Druck im Bereich von 400 bis 12000 bar, bevorzugt im Bereich 2500 bis 10000 bar, besonders bevorzugt im Bereich von 4000 bis 10000 bar, oder
- isostatischen Druck im Bereich von 50 bis 750 bar, bevorzugt im Bereich von 100 bis 600 bar, besonders bevorzugt im Bereich von 200 bis 500 bar.

19. Verfahren zur Herstellung eines dentalen Fräsrohlings nach einem der vorangehenden Aspekte, wobei das anorganische Füllstoffpulver F einen oder mehrere anorganische Füllstoffe umfasst, ausgewählt aus der Gruppe bestehend aus Bariumsilikatgläsern, Bariumaluminiumsilikatgläsern, Bariumborosilikatsilikatgläsern, Bariumboroaluminiumsilikatgläsern, Bariumborofluoroaluminiumsilikatgläsern, Strontiumsilikatgläsern, Strontiumaluminiumsilikatgläsern, Strontiumborosilikatgläsern, Strontiumboroaluminiumsilikatsilikatgläsern, Strontiumborofluoroaluminiumsilikatgläsern, Zirkonsilikatgläsern, Quarz, Cristobalit, pyrogener Kieselsäure und Ytterbiumfluorid, bevorzugt ausgewählt aus der Gruppe bestehend aus Bariumboroaluminiumsilikatsilikatgläsern, pyrogener Kieselsäure und Ytterbiumfluorid.

20. Verfahren zur Herstellung eines dentalen Fräsrohlings nach einem der vorangehenden Aspekte, wobei das anorganische Füllstoffpulver F einen D50-Wert von 0.18 bis 10 $\mu$m, vorzugsweise von 0.4 bis 5 $\mu$m, aufweist.

21. Verfahren zur Herstellung eines dentalen Fräsrohlings nach einem der vorangehenden Aspekte, wobei das anorganische Füllstoffpulver F ein erstes anorganisches Füllstoffpulver F1 und ein zweites anorganisches Füllstoffpulver F2 umfasst.

22. Verfahren zur Herstellung eines dentalen Fräsrohlings nach einem der vorangehenden Aspekte, wobei sich das erste anorganische Füllstoffpulver F1 und das zweite anorganische Füllstoffpulver F2 in ihrer chemischen Zusammensetzung und/oder in ihrer Partikelgröße unterscheiden.

23. Verfahren zur Herstellung eines dentalen Fräsrohlings nach einem der vorangehenden Aspekte, wobei das erste anorganische Füllstoffpulver F1 einen D50-Wert von 0.4 bis 1.0 $\mu$m, vorzugsweise von 0.5 bis 0.9 $\mu$m, und das zweite anorganische Füllstoffpulver F2 einen D50-Wert von 1.2 bis 5.0 $\mu$m, vorzugsweise von 1.5 bis 4.0 $\mu$m, aufweist.

24. Verfahren zur Herstellung eines dentalen Fräsrohlings nach einem der vorangehenden Aspekte, wobei das anorganische Füllstoffpulver (F) mindestens 50 Gew.-%, bevorzugt mindestens 70 Gew.-%, besonders bevorzugt mindestens 90 Gew.-% und ganz besonders bevorzugt 100 Gew.-% Bestandteile enthält, die in der Lage sind mit dem Silan PG-L-Si$(R^1)_n(OR^2)_{(3-n)}$ zu reagieren.

25. Verfahren zur Herstellung eines dentalen Fräsrohlings nach einem der vorangehenden Aspekte, wobei die polymerisierbare Gruppe PG des Silans PG-L-Si$(R^1)_n(OR^2)_{(3-n)}$ ausgewählt ist aus der Gruppe bestehend aus -OC(=O)CH=CH$_2$, -OC(=O)C(CH$_3$)=CH$_2$, -NHC(=O)CH=CH$_2$ und -NHC(=O)C(CH$_3$)=CH$_2$, bevorzugt -OC(=O)CH=CH$_2$ und -OC(=O)C(CH$_3$)=CH$_2$, besonders bevorzugt -OC(=O)C(CH$_3$)=CH$_2$.

26. Verfahren zur Herstellung eines dentalen Fräsrohlings nach einem der vorangehenden Aspekte, wobei die Linkergruppe L des Silans PG-L-Si$(R^1)_n(OR^2)_{(3-n)}$ eine Alkylengruppe mit 1 bis 12 C-Atomen ist, die von einem Sauerstoffatom oder einer Urethangruppe unterbrochen sein kann.

27. Verfahren zur Herstellung eines dentalen Fräsrohlings nach einem der vorangehenden Aspekte, wobei L ausgewählt ist aus der Gruppe bestehend aus -(CH$_2$)$_p$-, -(CH$_2$)$_q$-OC(=O)NH-(CH$_2$)$_p$- und -(CH$_2$)$_q$-NHC(=O)O-(CH$_2$)$_p$-, mit p = 1 bis 8 und q = 2 bis 4.

28. Verfahren zur Herstellung eines dentalen Fräsrohlings nach einem der vorangehenden Aspekte, wobei das Silan PG-L-Si$(R^1)_n(OR^2)_{(3-n)}$ ausgewählt ist aus der Gruppe bestehend aus H$_2$C=C(CH$_3$)C(=O)O-(CH$_2$)$_p$-Si(OR$^2$)$_3$, H$_2$C=C(CH$_3$)C(=O)O-(CH$_2$)$_q$-NHC(=O)O-(CH$_2$)$_p$-Si(OR$^2$)$_3$ und H$_2$C=C(CH$_3$)C(=O)O-(CH$_2$)$_q$-OC(=O)NH-(CH$_2$)$_p$-Si(OR$^2$)$_3$ worin R$^2$ eine C1-bis C4-Alkylgruppe ist, p = 1 bis 8 und q = 2 bis 4 ist.

29. Verfahren zur Herstellung eines dentalen Fräsrohlings nach einem der vorangehenden Aspekte, wobei das Silan PG-L-Si(R$^1$)$_n$(OR$^2$)$_{(3-n)}$ ausgewählt ist aus der Gruppe bestehend aus H$_2$C=C(CH$_3$)C(=O)O-(CH$_2$)-Si(OR$^2$)$_3$, H$_2$C=C(CH$_3$)C(=O)O-(CH$_2$)$_3$-Si(OR$^2$)$_3$, H$_2$C=C(CH$_3$)C(=O)O-(CH$_2$)$_2$-NHC(=O)O-(CH$_2$)$_3$-Si(OR$^2$)$_3$ und H$_2$C=C(CH$_3$)C(=O)O-(CH$_2$)$_2$-OC(=O)NH-(CH$_2$)$_3$-Si(OR$^2$)$_3$ worin R$^2$ eine C1- bis C4-Alkylgruppe ist.

30. Verfahren zur Herstellung eines dentalen Fräsrohlings nach einem der vorangehenden Aspekte, wobei das Silan PG-L-Si(R$^1$)$_n$(OR$^2$)$_{(3-n)}$ 3-Methacryloxypropyltrimethoxysilan ist.

31. Verfahren zur Herstellung eines dentalen Fräsrohlings nach einem der vorangehenden Aspekte, wobei die flüssige, polymerisierbare Zusammensetzung (M) umfasst

A) ein, zwei oder mehrere polymerisierbare Monomere, vorzugsweise (Meth)acrylate und/oder (Meth)acrylamide,
B) einen Initiator oder ein Initiatorsystem, bevorzugt ein thermischer Initiator,
C) optional anorganische, nicht-agglomerierte, nicht-aggregierte Partikel mit einer mittleren Partikelgröße von kleiner 80 nm und
D) optional Farbmittel.

32. Verfahren zur Herstellung eines dentalen Fräsrohlings nach einem der vorangehenden Aspekte, wobei die flüssige, polymerisierbare Zusammensetzung (M) umfasst

A) in einer Menge von 39 bis 99 Gew.-%, bevorzugt 39 bis 79 Gew.-%, besonders bevorzugt 39 bis 59 Gew.-%,
B) in einer Menge von 0.5 bis 5 Gew.-%, bevorzugt 0.5 bis 3.0 Gew.-%, besonders bevorzugt 1.0 bis 2.5 Gew.-%,
C) in einer Menge von 0 bis 60 Gew.-%, bevorzugt 20 bis 60 Gew.-%, besonders bevorzugt 40 bis 60 Gew.-% und
D) in einer Menge von 0 bis 1 Gew.-%, bevorzugt 0 bis 0.5 Gew.-%, besonders bevorzugt 0.0001 bis 0.1 Gew.-%,

jeweils bezogen auf die Gesamtmasse der flüssigen, polymerisierbaren Zusammensetzung (M).

33. Verfahren zur Herstellung eines dentalen Fräsrohlings nach einem der vorangehenden Aspekte, wobei der Anteil an polymerisierbaren Monomeren (A) 39 bis 99 Gew.-%, bevorzugt 39 bis 79 Gew.-%, besonders bevorzugt 39 bis 59 Gew.-%, bezogen auf die Gesamtmasse der flüssigen, polymerisierbaren Zusammensetzung (M) beträgt.

34. Verfahren zur Herstellung eines dentalen Fräsrohlings nach einem der vorangehenden Aspekte, wobei die polymerisierbaren Monomere (A) ein oder mehrere Momomere ausgewählt aus der Gruppe bestehend aus Ethylenglycoldi(meth)acrylat, Diethylenglycoldi(meth)acrylat, Triethylen-glycoldi(meth)acrylat, Tetraethylenglycoldi(meth)acrylat, Polyethylenglycol-di(meth)acrylat, Propylenglycoldi(meth)acrylat, Dipropylenglycoldi(meth)acrylat, Tripropylenglycoldi(meth)acrylat, Tetrapropylenglycol-di(meth)acrylat, Polypropylenglycoldi(meth)acrylat, Neopentylglycol-di(meth)acrylat, 1,4-Butandioldi(meth)acrylat, 1,6-Hexandiol¬di(meth)-acrylat, 1,9-Nonandioldi(meth)acrylat, 1,10-Decandioldi(meth)acrylat, 1,12-Dodecandioldi(meth)acrylat, Tricyclo[5.2.1.0$^{2,6}$]decan-3(4),8(9)-dimethanol-di(meth)acrylat, 2-Hydroxypropyl-1,3-di(meth)acrylat, 3-Hydroxypropyl-1,2-di(meth)acrylat, Urethandi(meth)acrylat, 7,7,9-Trimethyl-3,14-dioxa-4,13-dioxo-5,12-diazahexadecan-1,16-diyldi(meth)acrylat, 7,9,9-Trimethyl-3,14-dioxa-4,13-dioxo-5,12-diazahexadecan-1,16-diyldi(meth)acrylat, 1,5,5-Trimethyl-1-[(2-(meth)acryloyloxyethyl)carbamoylmethyl]-3-(2-(meth)acryl-oyloxyethyl)carbamoylcyclohexan, 1,3-Bis(3',8'-dioxo-2'-aza-4',7'-dioxa-decyl-9'-en)phenyl, 1,3-Bis(9'-methyl-3',8'-dioxo-2'-aza-4',7'-dioxa-decyl-9'-en)phenyl, 1,3-Bis(1',1'-dimethyl-3',8'-dioxo-2'-aza-4',7'-dioxa-decyl-9'-en)phenyl, 1,3-Bis(1',1',9'-trimethyl-3',8'-dioxo-2'-aza-4',7'-dioxa-decyl-9'-en)phenyl, 3(4),8(9)-Bis-(4',7'-dioxa-3',8'-dioxo-2'-aza-decyl-9'-en)tetrahydrodicyclopentadien, 3(4),8(9)-Bis-(4',7'-dioxa- 3',8'-dioxo-2'-aza-9'-methyl-decyl-9'-en)tetrahydrodicyclopentadien, Trimethylolpropandi-(meth)acrylat, Trimethylolpropantri(meth)acrylat, ethoxyliertes Trimethylol-propantri(meth)acrylat, Ditrimethylolpropantetra(meth)acrylat, ethoxyliertes Ditrimethylolpropantetra(meth)acrylat, Pentaerythritoldi(meth)acrylat, Penta-erythritoltri(meth)acrylat, Pentaerythritoltetra(meth)acrylat, Dipenta-erythritoltetra(meth)acrylat, Dipentaerythritolpenta(meth)acrylat, Dipenta-erythritolhexa(meth)acrylat, 2,2-Bis[4-3-(meth)acryloyloxy-2-hydroxy-propoxy]phenyl]propan, 2,2-Bis[4-[2-(meth)acryloyloxy-3-hydroxypropoxy]-phenyl]propan, ethoxyliertes Bisphenol-A-di(meth)acrylat, propoxyliertes Bisphenol-A-di(meth)acrylat, 2,2-Bis[4-(meth)acryloyloxyethoxyphenyl]-propan, 2,2-Bis[4-(meth)acryloyloxydiethoxyphenyl]propan, 2,2-Bis[4-(meth)acryloyloxytriethoxyphenyl]propan, 2,2-Bis[4-(meth)acryloyloxy-tetraethoxyphenyl]propan, 2,2-Bis[4-(meth)acryloyloxypentaethoxyphenyl]-propan, 2,2-Bis[4-(meth)acryloyloxydipropoxyphenyl]propan, 2,2-Bis[4-(meth)acryloyloxyethoxyphenyl]-2-[4-(meth)acryloyloxydiethoxyphenyl]-propan, 2-[4-(Meth)acryloyloxydiethoxyphenyl]-2-[4-(meth)acryloyloxy-triethoxyphenyl]propan, 2-[4-(Meth)acryloyloxdipropoxyphenyl]-2-[4-(meth)-acryloyloxytriethoxyphenyl]propan, 2-Hydroxyethyl(meth)acrylat, 2-Hydroxypropyl(meth)acrylat, 3-Hydroxypropyl(meth)acrylat, 1,2-Dihydroxypropyl-(meth)acrylat, 1,3-Dihydroxypropyl(meth)acrylat, Methyl(meth)acrylat, Ethyl(meth)acrylat, Propyl(meth)acrylat, Butyl(meth)acrylat, Hexyl(meth)-acrylat, 2-Ethylhexyl(meth)acrylat, Tetrahydrofurfuryl(meth)acrylat, Isobornyl(meth)acrylat, Lauryl(meth)acrylat, Cyclohexyl(meth)acrylat, (Octahydro-4,7-methano-1H-indenyl)methyl(meth)acrylat, Benzyl(meth)-acrylat und Phenoxyethyl(meth)acrylat, bevorzugt ausgewählt aus der Gruppe bestehend aus Triethylenglycoldi(meth)acrylat, Tetraethylen-glycoldi(meth)acrylat, 1,6-Hexandiol¬di(meth)acrylat, 1,10-Decandioldi-(meth)acrylat, 1,12-Dodecandioldi(meth)acrylat, Tricyclo[5.2.1.0$^{2,6}$]decan-3(4),8(9)-dimethanol-di(meth)acrylat, 2-Hydroxypropyl-1,3-di(meth)acrylat, 3-Hydroxypropyl-1,2-di(meth)acrylat, Urethandi(meth)acrylat, 7,7,9-Trimethyl-3,14-dioxa-4,13-dioxo-5,12-diazahexadecan-1,16-diyldi(meth)-acrylat, 7,9,9-Trimethyl-3,14-dioxa-4,13-dioxo-5,12-diazahexadecan-1,16-diyldi(meth)acrylat, 1,5,5-Trimethyl-1-[(2-(meth)acryloyloxyethyl)carbamoylmethyl]-3-(2-(meth)acryloyloxyethyl)carbamoylcyclohexan, 1,3-Bis(3',8'-dioxo-2'-aza-4',7'-dioxa-decyl-9'-en)phenyl, 1,3-Bis(9'-methyl-3',8'-dioxo-2'-aza-4',7'-dioxa-decyl-9'-en)phenyl, 1,3-Bis(1',1'-dimethyl-3',8'-dioxo-2'-aza-4',7'-dioxa-decyl-9'-en)phenyl, 1,3-Bis(1',1',9'-trimethyl-3',8'-dioxo-2'-aza-4',7'-dioxa-decyl-9'-en)phenyl, 3(4),8(9)-Bis-(4',7'-dioxa-3',8'-dioxo-2'-aza-decyl-9'-en)tetrahydrodicyclopentadien, 3(4),8(9)-Bis-(4',7'-dioxa- 3',8'-dioxo-2'-aza-9'-methyl-decyl-9'-en)tetrahydrodicyclopentadien, 2,2-Bis[4-[3-(meth)acryloyloxy-2-hydroxypropoxy]phenyl]propan, 2,2-Bis[4-[2-(meth)-acryloyloxy-3-hydroxypropoxy]phenyl]propan, ethoxyliertes Bisphenol-A-di(meth)acrylat, propoxyliertes Bisphenol-A-di(meth)acrylat, 2,2-Bis[4-(meth)acryloyloxyethoxyphenyl]propan, 2,2-Bis[4-(meth)acryloyl-oxydiethoxyphenyl]propan, 2,2-Bis[4-(meth)acryloyloxytriethoxyphenyl]-propan, 2,2-Bis[4-(meth)acryloyloxytetraethoxyphenyl]propan, 2,2-Bis[4-(meth)acryloyloxypentaethoxyphenyl]-propan, 2,2-Bis[4-(meth)acryloyloxy-dipropoxyphenyl]propan, 2,2-Bis[4-(meth)acryloyloxyethoxyphenyl]-2-[4-(meth)acryloyloxydiethoxyphenyl]propan, 2-[4-(Meth)acryloyloxydiethoxy-phenyl]-2-[4-(meth)acryloyloxytriethoxyphenyl]propan und 2-[4-(Meth)-acryloyloxdipropoxyphenyl]-2-[4-(meth)acryloyloxytriethoxyphenyl]propan, umfassen.

35. Verfahren zur Herstellung eines dentalen Fräsrohlings nach einem der vorangehenden Aspekte, wobei der Anteil an Initiatoren (B) 0.5 bis 5 Gew.-%, bevorzugt 0.5 bis 3.0 Gew.-%, besonders bevorzugt 1.0 bis 2.5 Gew.-%, bezogen auf die Gesamtmasse der flüssigen, polymerisierbaren Zusammensetzung (M) beträgt.

36. Verfahren zur Herstellung eines dentalen Fräsrohlings nach einem der vorangehenden Aspekte, wobei der Initiator oder das Initiatorsystem (B) ausgewählt ist aus der Gruppe bestehend aus Ketonperoxiden, Peroxyketalen, Hydroperoxiden, Dialkylperoxiden, Diacylperoxiden, Peroxyestern, Peroxydicarbonaten und Azoverbindungen.

37. Verfahren zur Herstellung eines dentalen Fräsrohlings nach einem der vorangehenden Aspekte, wobei der Initiator (B) ausgewählt ist aus der Gruppe bestehend aus Dibenzoylperoxid, Bis(2,4-dichlorbenzoyl)peroxid, Dilauroylperoxid, Dicumyl-peroxid, tert.-Butylperbenzoat, Cumolhydroperoxid, 3,5,5-Trimethylhexansäure-tert.-butylperoxyester 2,2'-Azobisisobutyronitril, 2,2'-Azobis(2-methylbutyronitril), 2,2'-Azobis-2,4-dimethyl-valeronitril, 2,2'-Azobis-1-cyclohexancabonitril und Dimethyl-2-2'-azobis-isobutyrat, bevorzugt ausgewählt aus der Gruppe bestehend aus Dibenzoylperoxid, Bis(2,4-dichlorbenzoyl)peroxid, Dilauroylperoxid und 2,2'-Azobisisobutyronitril, besonders bevorzugt Dibenzoylperoxid.

38. Verfahren zur Herstellung eines dentalen Fräsrohlings nach einem der vorangehenden Aspekte, wobei der Anteil an anorganischen, nicht-agglomerierten, nicht-aggregierten Partikeln (C) 0 bis 60 Gew.-%, bevorzugt 20 bis 60 Gew.-%, besonders bevorzugt 40 bis 60 Gew.-%, bezogen auf die Gesamtmasse der flüssigen, polymerisierbaren Zusammensetzung (M) beträgt.

39. Verfahren zur Herstellung eines dentalen Fräsrohlings nach einem der vorangehenden Aspekte, wobei die anorganischen, nicht-agglomerierten, nicht-aggregierten Partikel (C) eine mittels DLS bestimmte mittlere Partikelgröße mit einem volumengewichteten D50-Wert von kleiner 80 nm, bevorzugt 8 bis 80 nm, besonders bevorzugt von 10 bis 60 nm aufweisen.

40. Verfahren zur Herstellung eines dentalen Fräsrohlings nach einem der vorangehenden Aspekte, wobei die anorganischen, nicht-agglomerierten, nicht-aggregierten Partikel (C) monodispers und vollständig dispergiert vorliegen.

41. Verfahren zur Herstellung eines dentalen Fräsrohlings nach einem der vorangehenden Aspekte, wobei die anorganischen, nicht-agglomerierten, nicht-aggregierten Partikel (C) $SiO_2$- und/oder $YbF_3$-Partikel sind.

42. Verfahren zur Herstellung eines dentalen Fräsrohlings nach einem der vorangehenden Aspekte, wobei die anorganische, nicht-agglomerierte, nicht-aggregierte Partikel (C) $SiO_2$-Partikel sind und mit einem Silan der Formel

PG-L-Si(R$^1$)$_n$(OR$^2$)$_{(3-n)}$, vorzugsweise mit 3-Methacryloxypropyltrimethoxysilan, oberflächenbeschichtet sind.

43. Verfahren zur Herstellung eines dentalen Fräsrohlings nach einem der vorangehenden Aspekte, wobei die anorganischen, nicht-agglomerierten, nicht-aggregierten Partikel (C) YbF$_3$-Partikel sind und 10-[(2-Methylprop-2-enoyl)oxy]decyldihydrogenphosphat oberflächenbeschichtet sind.

44. Verfahren zur Herstellung eines dentalen Fräsrohlings nach einem der vorangehenden Aspekte, wobei der Anteil an Farbmitteln (D) 0 bis 1 Gew.-%, bevorzugt 0 bis 0.5 Gew.-%, besonders bevorzugt 0.0001 bis 0.1 Gew.-%, jeweils bezogen auf die Gesamtmasse der flüssigen, polymerisierbaren Zusammensetzung (M) beträgt.

45. Verfahren zur Herstellung eines dentalen Fräsrohlings nach einem der vorangehenden Aspekte, wobei die Farbmittel (D) anorganische Farbpigmente, organische Farbpigmente oder Farbstoffe sind.

46. Verfahren zur Herstellung eines dentalen Fräsrohlings nach einem der vorangehenden Aspekte, wobei die Farbmittel (D) anorganische Farbpigmente ausgewählt aus der Gruppe bestehend aus Eisenoxid, Titandioxid, Bariumsulfat und Aluminiumoxid umfassen.

47. Verfahren zur Herstellung eines dentalen Fräsrohlings nach einem der vorangehenden Aspekte, wobei die Farbmittel (D) organische Farbstoffe umfassen, die in der flüssigen, polymerisierbaren Zusammensetzung (M) gelöst werden und mit dieser in Schritt iii zugegeben werden.

48. Verfahren zur Herstellung eines dentalen Fräsrohlings nach einem der vorangehenden Aspekte, wobei die Farbmittel (D) anorganische oder organische Farbpigmente umfassen, die in der flüssigen, polymerisierbaren Zusammensetzung (M) dispergiert und mit dieser in Schritt iii zugegeben werden.

49. Verfahren zur Herstellung eines dentalen Fräsrohlings nach einem der vorangehenden Aspekte, wobei das Massenverhältnis von oberflächenbeschichtetem Pulver (F$_{sil}$) zur flüssigen, polymerisierbaren Zusammensetzung (M) im Bereich von 3:1 bis 10:1, bevorzugt im Bereich von 4:1 bis 9:1 und besonders bevorzugt im Bereich von 5:1 bis 8:1, liegt.

50. Verfahren zur Herstellung eines dentalen Fräsrohlings nach einem der vorangehenden Aspekte, wobei der Gesamtfüllstoffgehalt, die Summe der Massenanteile des oberflächenbeschichteten Pulvers (F$_{sil}$) und gegebenenfalls. der anorganischen, nicht-agglomerierten, nicht-aggregierten Partikel (C), im Bereich von 80 bis 95 Gew.-%, bevorzugt im Bereich von 83 bis 94 Gew.-% und besonders bevorzugt im Bereich von 85 bis 93 Gew.-%, jeweils bezogen auf die Gesamtmasse des Fräsrohlings liegt.

51. Dentaler Fräsrohling hergestellt in einem Verfahren nach einem der vorangehenden Aspekte.

52. Dentaler Fräsrohling hergestellt durch Verpressen und Polymerisation eines anorganischen Füllstoffpulvers (F$_{sil,ads}$), auf dessen Oberfläche sich

eine Beschichtung mit einem Silan (S) PG-L-Si(R$^1$)$_n$(OR$^2$)$_{(3-n)}$ worin PG eine polymerisierbare Gruppe ist, L eine Linkergruppe ist, die die polymerisierbare Gruppe PG an das Si-Atom bindet, R$^1$ eine C1- bis C4-Alkylgruppe oder Phenylgruppe ist, R$^2$ eine C1- bis C4-Alkylgruppe oder ein H-Atom ist und n = 0, 1 oder 2 ist, und
eine darauf adsorbierte flüssige, polymerisierbare Zusammensetzung (M) umfassend

(A) ein, zwei oder mehrere polymerisierbare Monomere, vorzugsweise (Meth)acrylate und/oder (Meth)acrylamide,
(B) einen Initiator oder ein Initiatorsystem, bevorzugt ein thermischer Initiator,
(C) optional anorganische, nicht-agglomerierte, nicht-aggregierte Partikel mit einer mittleren Partikelgröße von kleiner 80 nm und
(D) optional Farbmittel

befindet.

53. Kit umfassend mehrere Fräsrohlinge, hergestellt in einem Verfahren nach einem der Aspekte 1 bis 50, wobei die Fräsrohlinge

- die gleiche monochrome Farbe aufweisen oder
- unterschiedliche monochrome Farben aufweisen oder
- den gleichen schichtweisen Farbverlauf aufweisen oder
- den gleichen kontinuierlichen Farbverlauf aufweisen.

54. Kit umfassend

- einen oder mehrere Fräsrohlinge, hergestellt in einem Verfahren nach einem der Aspekte 1 bis 50,
- ein Konditionierungsmittel für aus den Fräsrohlingen gefertigte indirekte Restaurationen und
- ein Befestigungsmaterial zur Befestigung von aus den Fräsrohlingen gefertigte indirekte Restaurationen.

**Patentansprüche**

1. Verfahren zur Herstellung eines dentalen Fräsrohlings umfassend die folgenden Schritte

   i) Herstellen oder Bereitstellen eines anorganischen Füllstoffpulvers (F),
   ii) zumindest teilweises Beschichten der Oberfläche des anorganischen Füllstoffpulvers (F) durch Reaktion mit einem Silan (S) der Formel

   $PG\text{-}L\text{-}Si(R^1)_n(OR^2)_{(3\text{-}n)}$ worin
   PG eine polymerisierbare Gruppe ist,
   L eine Linkergruppe ist, die die polymerisierbare Gruppe PG an das Si-Atom bindet,
   $R^1$ eine C1- bis C4-Alkylgruppe oder Phenylgruppe ist,
   $R^2$ eine C1- bis C4-Alkylgruppe oder ein H-Atom ist und
   n = 0, 1 oder 2 ist,
   wobei ein oberflächenbeschichtetes Pulver ($F_{sil}$) erhalten wird,

   iii) Adsorbieren einer flüssigen, polymerisierbaren Zusammensetzung (M), die in der Lage ist mit der polymerisierbaren Gruppe PG zu polymerisieren, auf der Oberfläche des in Schritt ii erhaltenen Füllstoffpulvers ($F_{sil}$), wobei ein oberflächenadsorbiertes Pulver ($F_{sil,ads}$) erhalten wird,
   iv) Füllen des in Schritt iii erhaltenen Pulvers ($F_{sil,ads}$) in eine Form,
   v) Verpressen des Pulvers ($F_{sil,ads}$) in der Form unter Druck und
   vi) Polymerisation der polymerisierbaren Zusammensetzung M und der polymerisierbaren Gruppen PG.

2. Verfahren zur Herstellung eines dentalen Fräsrohlings nach Anspruch 1, wobei das Verfahren zusätzlich einen oder mehrere der folgenden Schritte umfasst

   vii) Entformen des in Schritt vi erhaltenen Fräsrohlings und/oder
   viii) Nachbearbeitung des in Schritt vi erhaltenen Fräsrohlings und/oder
   ix) Befestigung eines Pins auf der Oberfläche des Fräsrohlings zur Befestigung des Fräsrohlings in einer Fräsmaschine.

3. Verfahren zur Herstellung eines dentalen Fräsrohlings nach einem der vorangehenden Ansprüche, wobei das Verpressen in Schritt v, vorzugsweise uniaxial oder biaxial, bei einem Druck im Bereich von 400 bis 12000 bar, bevorzugt im Bereich von 2500 bis 10000 bar, besonders bevorzugt im Bereich von 4000 bis 10000 bar, erfolgt.

4. Verfahren zur Herstellung eines dentalen Fräsrohlings nach einem der vorangehenden Ansprüche, wobei die Polymerisation in Schritt vi, vorzugsweise isostatisch, bei einem Druck im Bereich von 50 bis 750 bar, bevorzugt im Bereich von 100 bis 600 bar, besonders bevorzugt im Bereich von 200 bis 500 bar, und/oder bei einer Temperatur im Bereich von 60 bis 160 °C, bevorzugt im Bereich von 80 bis 140 °C, erfolgt.

5. Verfahren zur Herstellung eines dentalen Fräsrohlings nach einem der vorangehenden Ansprüche, wobei die Schritte des Verpressens v und der Polymerisation vi gleichzeitig bei einer Temperatur im Bereich von 60 bis 160 °C, bevorzugt im Bereich von 80 bis 140 °C, erfolgen durch

   - uniaxialen oder biaxialen Druck im Bereich von 400 bis 12000 bar, bevorzugt im Bereich 2500 bis 10000 bar, besonders bevorzugt im Bereich von 4000 bis 10000 bar,

oder

- isostatischen Druck im Bereich von 50 bis 750 bar, bevorzugt im Bereich von 100 bis 600 bar, besonders bevorzugt im Bereich von 200 bis 500 bar.

6. Verfahren zur Herstellung eines dentalen Fräsrohlings nach einem der vorangehenden Ansprüche, wobei die polymerisierbare Gruppe PG des Silans (S) ausgewählt ist aus der Gruppe bestehend aus -OC(=O)CH=CH$_2$, -OC(=O)C(CH$_3$)=CH$_2$, -NHC(=O)CH=CH$_2$ und -NHC(=O)C(CH$_3$)=CH$_2$, bevorzugt -OC(=O)CH=CH$_2$ und -OC(=O)C(CH$_3$)=CH$_2$.

7. Verfahren zur Herstellung eines dentalen Fräsrohlings nach einem der vorangehenden Ansprüche, wobei Linkergruppe L des Silans (S) eine Alkylengruppe mit 1 bis 12 C-Atomen, die von einem Sauerstoffatom oder einer Urethangruppe unterbrochen sein kann, bevorzugt eine Gruppe ausgewählt aus der Gruppe bestehend aus -(CH$_2$)$_p$-, -(CH$_2$)$_q$-OC(=O)NH-(CH$_2$)$_p$-und-(CH$_2$)$_q$-NHC(=O)O-(CH$_2$)$_p$-, mit p = 1 bis 8 und q = 2 bis 4 ist.

8. Verfahren zur Herstellung eines dentalen Fräsrohlings nach einem der vorangehenden Ansprüche, wobei das anorganische Füllstoffpulvers Feinen oder mehrere anorganische Füllstoffe umfasst, ausgewählt aus der Gruppe bestehend aus Bariumsilikatgläsern, Bariumaluminiumsilikatgläsern, Bariumborosilikatsilikatgläsern, Bariumboroaluminiumsilikatgläsern, Bariumborofluoroaluminiumsilikatgläsern, Strontiumsilikatgläsern, Strontiumaluminiumsilikatgläsern, Strontiumborosilikatgläsern, Strontiumboroaluminiumsilikatsilikatgläsern, Strontiumborofluoroaluminiumsilikatgläsern, Zirkonsilikatgläsern, Quarz, Cristobalit, pyrogener Kieselsäure und Ytterbiumfluorid, bevorzugt ausgewählt aus der Gruppe bestehend aus Bariumboroaluminiumsilikatsilikatgläsern, pyrogener Kieselsäure und Ytterbiumfluorid.

9. Verfahren zur Herstellung eines dentalen Fräsrohlings nach einem der vorangehenden Ansprüche, wobei das anorganische Füllstoffpulver F einen D50-Wert von 0.18 bis 10 $\mu$m, vorzugsweise von 0.4 bis 5 $\mu$m, aufweist.

10. Verfahren zur Herstellung eines dentalen Fräsrohlings nach einem der vorangehenden Ansprüche, wobei das anorganische Füllstoffpulver F ein erstes anorganisches Füllstoffpulver F1 und ein zweites anorganisches Füllstoffpulver F2 umfasst.

11. Verfahren zur Herstellung eines dentalen Fräsrohlings nach einem der vorangehenden Ansprüche, wobei das erste anorganische Füllstoffpulver F1 einen D50-Wert von 0.4 bis 1.0 $\mu$m, vorzugsweise von 0.5 bis 0.9 $\mu$m, und das zweite anorganische Füllstoffpulver F2 einen D50-Wert von 1.2 bis 5.0 $\mu$m, vorzugsweise von 1.5 bis 4.0 $\mu$m, aufweist.

12. Verfahren zur Herstellung eines dentalen Fräsrohlings nach einem der vorangehenden Ansprüche, wobei die flüssige, polymerisierbare Zusammensetzung (M) umfasst

A) ein, zwei oder mehrere polymerisierbare Monomere, vorzugsweise (Meth)acrylate und/oder (Meth)acrylamide,
B) einen Initiator oder ein Initiatorsystem, bevorzugt ein thermischer Initiator,
C) optional anorganische, nicht-agglomerierte, nicht-aggregierte Partikel mit einer mittleren Partikelgröße von kleiner 80 nm und
D) optional Farbmittel,

und/oder

A) in einer Menge von 39 bis 99 Gew.-%, bevorzugt 39 bis 79 Gew.-%, besonders bevorzugt 39 bis 59 Gew.-%,
B) in einer Menge von 0.5 bis 5 Gew.-%, bevorzugt 0.5 bis 3.0 Gew.-%, besonders bevorzugt 1.0 bis 2.5 Gew.-%,
C) in einer Menge von 0 bis 60 Gew.-%, bevorzugt 20 bis 60 Gew.-%, besonders bevorzugt 40 bis 60 Gew.-% und
D) in einer Menge von 0 bis 1 Gew.-%, bevorzugt 0 bis 0.5 Gew.-%, besonders bevorzugt 0.0001 bis 0.1 Gew.-%,

jeweils bezogen auf die Gesamtmasse der flüssigen, polymerisierbaren Zusammensetzung (M).

13. Verfahren zur Herstellung eines dentalen Fräsrohlings nach einem der vorangehenden Ansprüche, wobei die polymerisierbaren Monomere (A) ein oder mehrere Momomere ausgewählt aus der Gruppe bestehend aus Ethylenglycoldi(meth)acrylat, Diethylenglycoldi(meth)acrylat, Triethylen-glycoldi(meth)acrylat, Tetraethylenglycoldi(meth)acrylat, Polyethylenglycol-di(meth)acrylat, Propylenglycoldi(meth)acrylat, Dipropylenglycol-di(meth)acrylat, Tripropylenglycoldi(meth)acrylat, Tetrapropylenglycol-di(meth)acrylat, Polypropylenglycol-di(meth)acrylat, Neopentylglycol-di(meth)acrylat, 1,4-Butandioldi(meth)acrylat, 1,6-Hexandiol¬di(meth)-acrylat, 1,9-Nonandioldi(meth)acrylat, 1,10-Decandioldi(meth)acrylat, 1,12-Dodecandioldi(meth)acrylat, Tricyclo[5.2.1.0$^{2,6}$]decan-3(4),8(9)-dimethanol-di(meth)acrylat, 2-Hydroxypropyl-1,3-di(meth)acrylat, 3-Hydroxypropyl-1,2-di(meth)acrylat, Urethandi(meth)acrylat, 7,7,9-Trimethyl-3,14-dioxa-4,13-dioxo-5,12-diazahexadecan-1,16-diyldi(meth)acrylat, 7,9,9-Trimethyl-3,14-dioxa-4,13-dioxo-5,12-diazahexadecan-1,16-diyldi(meth)acrylat, 1,5,5-Trimethyl-1-[(2-(meth)acryloyloxyethyl)carbamoylmethyl]-3-(2-(meth)acryl-oyloxyethyl)carbamoylcyclohexan, 1,3-Bis(3',8'-dioxo-2'-aza-4',7'-dioxa-decyl-9'-en)phenyl, 1,3-Bis(9'-methyl-3',8'-dioxo-2'-aza-4',7'-dioxa-decyl-9'-en)phenyl, 1,3-Bis(1',1'-dimethyl-3',8'-dioxo-2'-aza-4',7'-dioxa-decyl-9'-en)phenyl, 1,3-Bis(1',1',9'-trimethyl-3',8'-dioxo-2'-aza-4',7'-dioxa-decyl-9'-en)phenyl, 3(4),8(9)-Bis-(4',7'-dioxa-3',8'-dioxo-2'-aza-decyl-9'-en)tetrahydrodicyclopentadien, 3(4),8(9)-Bis-(4',7'-dioxa- 3',8'-dioxo-2'-aza-9'-methyl-decyl-9'-en)tetrahydrodicyclopentadien, Trimethylolpropandi-(meth)acrylat, Trimethylolpropantri(meth)acrylat, ethoxyliertes Trimethylol-propantri(meth)acrylat, Ditrimethylolpropantetra(meth)acrylat, ethoxyliertes Ditrimethylolpropantetra(meth)acrylat, Pentaerythritoldi(meth)acrylat, Penta-erythritoltri(meth)acrylat, Pentaerythritoltetra(meth)acrylat, Dipenta-erythritoltetra(meth)acrylat, Dipentaerythritolpenta(meth)acrylat, Dipenta-erythritolhexa(meth)acrylat, 2,2-Bis[4-[3-(meth)acryloyloxy-2-hydroxy-propoxy]phenyl]propan, 2,2-Bis[4-[2-(meth)acryloyloxy-3-hydroxypropoxy]-phenyl]propan, ethoxyliertes Bisphenol-A-di(meth)acrylat, propoxyliertes Bisphenol-A-di(meth)acrylat, 2,2-Bis[4-(meth)acryloyloxyethoxyphenyl]-propan, 2,2-Bis[4-(meth)acryloyloxydiethoxyphenyl]propan, 2,2-Bis[4-(meth)acryloyloxytriethoxyphenyl]propan, 2,2-Bis[4-(meth)acryloyloxy-tetraethoxyphenyl]propan, 2,2-Bis[4-(meth)acryloyloxypentaethoxyphenyl]-propan, 2,2-Bis[4-(meth)acryloyloxydipropoxyphenyl]propan, 2,2-Bis[4-(meth)acryloyloxyethoxyphenyl]-2-[4-(meth)acryloyloxydiethoxyphenyl]-propan, 2-[4-(Meth)acryloyloxydiethoxyphenyl]-2-[4-(meth)acryloyloxy-triethoxyphenyl]propan, 2-[4-(Meth)acryloyloxdipropoxyphenyl]-2-[4-(meth)-acryloyloxytriethoxyphenyl]propan, 2-Hydroxyethyl(meth)acrylat, 2-Hydroxypropyl(meth)acrylat, 3-Hydroxypropyl(meth)acrylat, 1,2-Dihydroxypropyl-(meth)acrylat, 1,3-Dihydroxypropyl(meth)acrylat, Methyl(meth)acrylat, Ethyl(meth)acrylat, Propyl(meth)acrylat, Butyl(meth)acrylat, Hexyl(meth)-acrylat, 2-Ethylhexyl(meth)acrylat, Tetrahydrofurfuryl(meth)acrylat, Isobornyl(meth)acrylat, Lauryl(meth)acrylat, Cyclohexyl(meth)acrylat, (Octahydro-4,7-methano-1H-indenyl)methyl(meth)acrylat, Benzyl(meth)-acrylat und Phenoxyethyl(meth)acrylat, bevorzugt ausgewählt aus der Gruppe bestehend aus Triethylenglycoldi(meth)acrylat, Tetraethylen-glycoldi(meth)acrylat, 1,6-Hexandiol¬di(meth)acrylat, 1,10-Decandioldi-(meth)acrylat, 1,12-Dodecandioldi(meth)acrylat, Tricyclo[5.2.1.0$^{2,6}$]decan-3(4),8(9)-dimethanol-di(meth)acrylat, 2-Hydroxypropyl-1,3-di(meth)acrylat, 3-Hydroxypropyl-1,2-di(meth)acrylat, Urethandi(meth)acrylat, 7,7,9-Trimethyl-3,14-dioxa-4,13-dioxo-5,12-diazahexadecan-1,16-diyldi(meth)-acrylat, 7,9,9-Trimethyl-3,14-dioxa-4,13-dioxo-5,12-diazahexadecan-1,16-diyldi(meth)acrylat, 1,5,5-Trimethyl-1-[(2-(meth)acryloyloxyethyl)carbamoylmethyl]-3-(2-(meth)acryloyloxyethyl)carbamoylcyclohexan, 1,3-Bis(3',8'-dioxo-2'-aza-4',7'-dioxa-decyl-9'-en)phenyl, 1,3-Bis(9'-methyl-3',8'-dioxo-2'-aza-4',7'-dioxa-decyl-9'-en)phenyl, 1,3-Bis(1',1'-dimethyl-3',8'-dioxo-2'-aza-4',7'-dioxa-decyl-9'-en)phenyl, 1,3-Bis(1', 1',9'-trimethyl-3',8'-dioxo-2'-aza-4',7'-dioxa-decyl-9'-en)phenyl, 3(4),8(9)-Bis-(4',7'-dioxa-3',8'-dioxo-2'-aza-decyl-9'-en)tetrahydrodicyclopentadien, 3(4),8(9)-Bis-(4',7'-dioxa- 3',8'-dioxo-2'-aza-9'-methyl-decyl-9'-en)tetrahydrodicyclopentadien, 2,2-Bis[4-[3-(meth)acryloyloxy-2-hydroxypropoxy]phenyl]propan, 2,2-Bis[4-[2-(meth)-acryloyloxy-3-hydroxypropoxy]phenyl]propan, ethoxyliertes Bisphenol-A-di(meth)acrylat, propoxyliertes Bisphenol-A-di(meth)acrylat, 2,2-Bis[4-(meth)acryloyloxyethoxyphenyl]propan, 2,2-Bis[4-(meth)acryloyl-oxydiethoxyphenyl]propan, 2,2-Bis[4-(meth)acryloyloxytriethoxyphenyl]-propan, 2,2-Bis[4-(meth)acryloyloxytetraethoxyphenyl]propan, 2,2-Bis[4-(meth)acryloyloxypentaethoxyphenyl]-propan, 2,2-Bis[4-(meth)acryloyloxy-dipropoxyphenyl]propan, 2,2-Bis[4-(meth)acryloyloxyethoxyphenyl]-2-[4-(meth)acryloyloxydiethoxyphenyl]propan, 2-[4-(Meth)acryloyloxydiethoxy-phenyl]-2-[4-(meth)acryloyloxytriethoxyphenyl]propan und 2-[4-(Meth)-acryloyloxdipropoxyphenyl]-2-[4-(meth)acryloyloxytriethoxyphenyl]propan, umfassen.

14. Verfahren zur Herstellung eines dentalen Fräsrohlings nach einem der vorangehenden Ansprüche, wobei der Initiator (B) ausgewählt ist aus der Gruppe bestehend aus Dibenzoylperoxid, Bis(2,4-dichlorbenzoyl)peroxid, Dilauroylperoxid, Dicumyl-peroxid, tert.-Butylperbenzoat, Cumolhydroperoxid, 3,5,5-Trimethylhexansäure-*tert*.-butylperoxyester 2,2'-Azobisisobutyronitril, 2,2'-Azobis(2-methylbutyronitril), 2,2'-Azobis-2,4-dimethyl-valeronitril, 2,2'-Azobis-1-cyclohexancabonitril und Dimethyl-2-2'-azobis-isobutyrat, bevorzugt ausgewählt aus der Gruppe bestehend aus Dibenzoylperoxid, Bis(2,4-dichlorbenzoyl)peroxid, Dilauroylperoxid und 2,2'-Azobisisobutyronitril, besonders bevorzugt Dibenzoylperoxid.

15. Verfahren zur Herstellung eines dentalen Fräsrohlings nach einem der vorangehenden Ansprüche, wobei die Farbmittel (D) ausgewählt sind aus der Gruppe bestehend aus anorganischen Farbpigmenten, organischen Farbpigmenten und Farbstoffen, und/oder wobei Farbmittel (D) anorganischen Farbpigmenten ausgewählt aus der Gruppe bestehend aus Eisenoxid, Titandioxid, Bariumsulfat und Aluminiumoxid umfassen.

**16.** Verfahren zur Herstellung eines dentalen Fräsrohlings nach einem der vorangehenden Ansprüche, wobei in einem der Schritte i, ii oder iii ein oder mehrere Farbmittel zugegeben werden oder wobei die flüssige, polymerisierbare Zusammensetzung (M) Farbmittel (D) umfasst.

**17.** Verfahren zur Herstellung eines dentalen Fräsrohlings nach einem der vorangehenden Ansprüche,

bei dem die Schritte i bis iv schichtweise wiederholt werden und die Pulverschichten in Schritt v gemeinsam verpresst werden oder bei dem die Schritte i bis v schichtweise wiederholt werden, wobei jeweils die einzelnen Schichten durch unterschiedliche Farbmittel und/oder durch eine unterschiedliche Menge an Farbmitteln unterschiedlich eingefärbt sind,
und/oder
bei dem parallel jeweils in den Schritten i bis iii zwei unterschiedlich gefärbte oberflächenadsorbierte Pulver $F_{sil,ads,1}$ und $F_{sil,ads,2}$ hergestellt werden und in Schritt iv unter Mischen in eine Form abgefüllt werden, wobei das Mischungsverhältnis von $F_{sil,ads,1}$ zu $F_{sil,ads,2}$ während des Abfüllen geändert, bevorzugt kontinuierlich geändert, wird.

**18.** Dentaler Fräsrohling hergestellt durch Verpressen und Polymerisation eines anorganischen Füllstoffpulvers ($F_{sil,ads}$), auf dessen Oberfläche sich

eine Beschichtung mit einem Silan (S) $PG-L-Si(R^1)_n(OR^2)_{(3-n)}$ worin PG eine polymerisierbare Gruppe ist, L eine Linkergruppe ist, die die polymerisierbare Gruppe PG an das Si-Atom bindet, $R^1$ eine C1- bis C4-Alkylgruppe oder Phenylgruppe ist, $R^2$ eine C1- bis C4-Alkylgruppe oder ein H-Atom ist und n = 0, 1 oder 2 ist,
und
eine darauf adsorbierte flüssige, polymerisierbare Zusammensetzung (M) umfassend

(A) ein, zwei oder mehrere polymerisierbare Monomere, vorzugsweise (Meth)acrylate und/oder (Meth)acrylamide,
(B) einen Initiator oder ein Initiatorsystem, bevorzugt ein thermischer Initiator,
(C) optional anorganische, nicht-agglomerierte, nicht-aggregierte Partikel mit einer mittleren Partikelgröße von kleiner 80 nm und
(D) optional Farbmittel

befindet.

Europäisches
Patentamt

European
Patent Office

Office européen
des brevets

**EUROPÄISCHER RECHERCHENBERICHT**

Nummer der Anmeldung

**EP 23 18 0585**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 112 494 341 A (SHENZHEN UPCERA BIOLOGICAL MAT CO LTD) 16. März 2021 (2021-03-16) * Absätze [0001], [0004], [0039] – [0045], [0073] – [0081], [0085], [0098], [0104] – [0106], [0110], [0111], [0115] * * Beispiel 1 * | 1-18 | INV. A61K6/16 A61K6/17 A61K6/64 A61K6/76 A61K6/77 A61K6/78 A61K6/887 |
| X | ----- CN 113 730 264 A (AIDITE QINHUANGDAO TECH CO LTD) 3. Dezember 2021 (2021-12-03) * das ganze Dokument * | 1-18 | |
| X,P | -& EP 4 144 334 A1 (AIDITE QINHUANGDAO TECH CO LTD [CN]) 8. März 2023 (2023-03-08) * Absätze [0001], [0002], [0010], [0011], [0016] – [0018], [0024] – [0027], [0029], [0030], [0033], [0036], [0037], [0044], [0045] * * Beispiel 1 * | 1-18 | |
| X | ----- DE 10 2018 103415 A1 (VOCO GMBH [DE]) 22. August 2019 (2019-08-22) | 18 | **RECHERCHIERTE SACHGEBIETE (IPC)** A61C A61K |
| A | * Absätze [0001], [0049], [0074], [0075], [0102], [0107], [0112] – [0114] * * Tabelle 1 * ----- | 1-17 | |

Der vorliegende Recherchenbericht wurde für alle Patentansprüche erstellt

| Recherchenort | Abschlußdatum der Recherche | Prüfer |
|---|---|---|
| Den Haag | 6. Dezember 2023 | Grave, Christian |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 23 18 0585

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-12-2023

| Im Recherchenbericht angeführtes Patentdokument | Datum der Veröffentlichung | Mitglied(er) der Patentfamilie | Datum der Veröffentlichung |
|---|---|---|---|
| CN 112494341 A | 16-03-2021 | KEINE | |
| CN 113730264 A | 03-12-2021 | CN 113730264 A | 03-12-2021 |
| | | EP 4144334 A1 | 08-03-2023 |
| | | JP 2023035918 A | 13-03-2023 |
| | | KR 20230033586 A | 08-03-2023 |
| | | US 2023091200 A1 | 23-03-2023 |
| EP 4144334 A1 | 08-03-2023 | CN 113730264 A | 03-12-2021 |
| | | EP 4144334 A1 | 08-03-2023 |
| | | JP 2023035918 A | 13-03-2023 |
| | | KR 20230033586 A | 08-03-2023 |
| | | US 2023091200 A1 | 23-03-2023 |
| DE 102018103415 A1 | 22-08-2019 | DE 102018103415 A1 | 22-08-2019 |
| | | EP 3527166 A1 | 21-08-2019 |
| | | US 2019247168 A1 | 15-08-2019 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69922413 T2
- EP 3050533 A1
- DE 2462271 C2
- EP 2623065 B1
- WO 2011087832 A1
- WO 2016140950 A1
- US 6345984 B2
- US 4544359 A
- EP 3363423 B1
- EP 3685798 B1
- EP 3287118 A1
- EP 2881077 A1
- JP 2017109036 A
- JP 2019098073 A
- JP 2019098074 A

**In der Beschreibung aufgeführte Nicht-Patentliteratur**

- *CHEMICAL ABSTRACTS,* 1565-94-2
- *CHEMICAL ABSTRACTS,* 41637-38-1
- *CHEMICAL ABSTRACTS,* 43048-08-4
- *CHEMICAL ABSTRACTS,* 109-16-0
- *CHEMICAL ABSTRACTS,* 6606-59-3
- *CHEMICAL ABSTRACTS,* 2530-85-0
- *CHEMICAL ABSTRACTS,* 122749-49-9
- *CHEMICAL ABSTRACTS,* 94-36-0
- *CHEMICAL ABSTRACTS,* 75980-60-8
- *CHEMICAL ABSTRACTS,* 128-37-0
- *CHEMICAL ABSTRACTS,* 3077-12-1